(19) Europäisches Patentamt
European Patent Office
Office européen des brevets

(11) **EP 2 105 843 B1**

(12) **FASCICULE DE BREVET EUROPEEN**

(45) Date de publication et mention
de la délivrance du brevet:
**16.03.2011 Bulletin 2011/11**

(51) Int Cl.:
*G06F 17/00* (2006.01)       *A61B 5/0464* (2006.01)
*A61N 1/362* (2006.01)       *A61N 1/39* (2006.01)
*A61B 5/04* (2006.01)

(21) Numéro de dépôt: **09290016.6**

(22) Date de dépôt: **09.01.2009**

(54) **Dispositif médical actif comprenant des moyens perfectionnés de discrimination entre tachycardies d'orgine ventriculaire et tachycardies d'orgine supraventriculaire**

Aktives Medizingerät, das verbesserte Mittel zur Unterscheidung zwischen Tachykardien ventrikulären Ursprungs und Tachykardien supraventrikulären Ursprungs enthält

Active medical device comprising perfected means for distinguishing between tachycardia with ventricular causes and tachycardia with supraventricular causes

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **28.03.2008 FR 0801691**

(43) Date de publication de la demande:
**30.09.2009 Bulletin 2009/40**

(73) Titulaire: **Ela Medical**
**92541 Montrouge (FR)**

(72) Inventeurs:
• **Dal Molin, Renzo**
  **92320 Châtillon (FR)**
• **Henry, Christine**
  **75014 Paris (FR)**

• **El Arab, Jinan**
  **75015 Paris (FR)**
• **Bouchet, Paola**
  **75014 Paris (FR)**
• **Dubois, Rémi**
  **75015 Paris (FR)**
• **Dreyfus, Gérard**
  **91190 Gif sur Yvette (FR)**

(74) Mandataire: **Dupuis-Latour, Dominique et al SEP Bardehle Pagenberg Dost Altenburg Geissler**
**10, boulevard Haussmann**
**75009 Paris (FR)**

(56) Documents cités:
WO-A-00/69517         WO-A-2006/039693
US-A1- 2003 083 587   US-A1- 2005 159 781
US-B1- 7 149 569

**Description**

L'invention concerne une technique d'analyse des tachyarythmies ventriculaires.

Elle est notamment applicable aux dispositifs médicaux implantables actifs (au sens de la directive 90/385/CEE du 20 juin 1990 du Conseil des communautés européennes), et plus particulièrement aux appareils permettant d'appliquer au coeur des thérapies impliquant la délivrance contrôlée d'impulsions électriques de haute énergie destinées à mettre fin à une tachyarythmie, et/ou des thérapies par stimulation à haute fréquence dites ATP (*AntiTachycardia Pacing*).

On notera cependant que l'invention peut être mise en oeuvre non seulement au sein d'un implant, mais également à l'extérieur du corps du patient, par exemple dans un programmateur externe utilisé par un praticien pour télécharger et analyser les signaux cardiaques recueillis et mémorisés par l'implant. L'invention peut également être mise en oeuvre dans un moniteur de "home monitoring", qui est un type particulier de programmateur dont le fonctionnement est entièrement automatisé ; un tel moniteur ne nécessite pas le recours à un praticien, et permet notamment de télétransmettre à intervalles réguliers à un site distant des données recueillies par un implant, pour analyse et suivi du patient. L'invention peut être également mise en oeuvre au niveau du serveur de données de ce site, à partir des données transmises telles quelles par le moniteur du patient.

De façon générale une tachyarythmie (un rythme cardiaque rapide anormal) peut être d'origine sinusale, auriculaire ou ventriculaire.

Mais il n'est pas toujours simple de déterminer l'origine d'une tachycardie avérée. Or, dans le cas d'un appareil susceptible par exemple d'appliquer une thérapie telle que la délivrance d'un choc de défibrillation, un tel choc ne doit être délivré que dans le cas d'une véritable tachycardie ventriculaire (TV), et non d'une tachycardie supraventriculaire (TSV). En effet, dans ce dernier cas, la tachycardie est d'origine auriculaire, et le choc qui serait délivré serait sans effet puisque l'électrode de défibrillation ne se trouve pas dans cette région.

Or l'application d'un choc de défibrillation chez un patient conscient est extrêmement douloureuse et angoissante, les énergies appliquées étant en effet très au-delà du seuil de douleur. En outre, l'application d'un choc de défibrillation n'est pas dénuée d'effets secondaires sur le rythme cardiaque (risque d'apparition de troubles secondaires), sur l'intégrité fonctionnelle du myocarde, et de façon générale sur l'équilibre physiologique du patient.

Il est donc important de ne délivrer de tels chocs que de façon appropriée et seulement si une autre thérapie moins douloureuse, telle qu'une stimulation appropriée de l'oreillette, n'est pas envisageable.

Plus précisément, une tachycardie peut recouvrir diverses formes de troubles du rythme cardiaque : lorsque l'on se trouve en présence d'une tachyarythmie, celle-ci peut avoir pour cause une fibrillation ventriculaire (FV), une tachycardie ventriculaire (TV), une tachycardie sinusale (TS) ou une tachycardie supraventriculaire (TSV). La TSV recouvre elle-même la tachycardie auriculaire, le flutter auriculaire et la fibrillation auriculaire (FA). Ces troubles peuvent d'ailleurs se superposer et l'on parle alors de "bitachycardie", notamment en présence d'une fibrillation auriculaire combinée à une tachycardie ventriculaire.

Toute la difficulté vient du fait que, dans bon nombre de situations pathologiques, certains événements pourtant présents ne sont pas visibles, car masqués par d'autres événements concomitants. Ainsi, les complexes larges d'une TV rapide rendent souvent difficile la reconnaissance des ondes P, ce qui ne permet pas toujours de les différencier de ceux d'un flutter associé à un bloc de branche fonctionnel.

La reconnaissance de ces phénomènes masqués, et des ondes P en particulier, est donc un élément fondamental dans ce domaine.

Or, si cette reconnaissance est difficile pour le clinicien, elle l'est encore plus pour les systèmes d'analyse automatique du rythme cardiaque. Les critères de discrimination employés par ces dispositifs comprennent en particulier la fréquence ventriculaire, la stabilité des intervalles ventriculaires (intervalles RR), l'analyse de l'association auriculo-ventriculaire (révélée par la stabilité de l'intervalle PR) et le mode de démarrage des tachycardies (présence d'une accélération brusque et cavité d'origine, ventriculaire ou auriculaire).

On pourra notamment se référer au EP 0 626 182 A1 (ELA Medical), qui décrit un algorithme de détection et de classification des tachyarythmies dénommé PARAD/PARAD+ mis en oeuvre en particulier dans les modèles *Defender* et *Ovatio* d'ELA Medical. Les EP 0 838 235 A1, EP 0 813 888 A1 et EP 1 208 873 A1 (tous trois au nom d'ELA Medical) décrivent divers perfectionnements à cet algorithme, permettant d'améliorer encore la discrimination entre TV et TSV, notamment pour éviter des faux diagnostics positifs (indication d'une TV alors qu'il s'agissait d'une TSV) ou négatifs (indication d'une TSV alors qu'il s'agissait d'une TV).

D'autres propositions ont également été formulées pour opérer la discrimination entre TV et TSV à partir d'une analyse morphologique du complexe QRS seul, ne faisant donc pas intervenir l'onde P difficile à reconnaître.

Ces techniques basées sur une analyse morphologique du QRS sont le plus souvent utilisées par les cardiologues en pratique clinique, lorsqu'ils analysent un tracé-ECG pour caractériser les arythmies ventriculaires qui sont généralement les plus menaçantes.

Mais l'application de ces méthodes à des algorithmes de détection automatique pour prothèse cardiaques implantées n'est pas suffisamment fiable, en partie parce que l'information potentielle contenue dans les signaux d'électrogramme endocavitaire (EGM) recueillis par ces prothèses n'est pas complètement maîtrisée, en tout état de cause beaucoup moins que les signaux d'électrocardiogramme (ECG) recueillis par un enregistreur externe. En particulier, les paramètres de normalité de ces signaux sont largement méconnus, ce qui ne permet pas de distinguer par comparaison les situations pathologiques de celles qui ne le sont pas.

S'y ajoute le fait que les algorithmes d'analyse, complexes, impliquent souvent, en termes de puissance de calcul et surtout de consommation énergétique, des moyens incompatibles avec ce qui est disponible à l'intérieur d'un dispositif miniature implanté. Ceci conduit à proposer des solutions algorithmiques sous-optimales qui ne permettent pas d'établir un diagnostic d'une fiabilité suffisante.

Il existe aujourd'hui différents algorithmes pour défibrillateurs implantables, basés sur une analyse morphologique.

Ces algorithmes mettent en oeuvre des méthodes basées sur la propriété suivante : lors d'un épisode de TSV, les impulsions électriques empruntent dans les ventricules les mêmes voies de conduction qu'en rythme sinusal, de sorte que la morphologie du signal de contraction ventriculaire est très similaire à celle du signal enregistré en rythme sinusal. A l'inverse, pendant un épisode de TV, les voies de conductions étant différentes, le signal électrique enregistré est différent.

Ces méthodes proposent donc d'effectuer la discrimination TV/TSV à partir d'une mesure de la ressemblance des signaux enregistrés pendant l'arythmie à ceux enregistrés en rythme sinusal.

Le US 2005/0159781 A1 (Cardiac Pacemakers, Inc.) décrit une technique dénommée "VTC" (*electrogram Vector Timing and Correlation*), où un algorithme analyse l'amplitude et la position temporelle d'un certain nombre de points singuliers, représentatifs, d'un complexe QRS recueilli sur une voie EGM endocavitaire, typiquement sur le ventricule droit (VD). Au préalable, l'algorithme crée un battement de référence en rythme sinusal, par les étapes consistant à : (i) recueillir un certain nombre de complexes d'un signal VD unipolaire (entre le boîtier et une électrode sur la sonde), (ii) aligner ces complexes entre eux à l'aide du signal VD bipolaire correspondant (recueilli entre deux électrodes sur la sonde), (iii) moyenner les complexes ainsi alignés, et enfin (iv) extraire du battement de référence moyen huit points représentatifs (minimum, maximum, points d'inflexion, etc.) pour définir un modèle ou *"template"*. Ultérieurement, lorsqu'une arythmie est détectée, l'algorithme VTC calcule le coefficient de corrélation entre ces huit points de référence du modèle et les huit points analogues du battement en tachycardie recueilli sur la (seule) voie du signal VD unipolaire. Si pour une tachycardie donnée l'algorithme trouve un nombre suffisant de battements non corrélés, alors cette tachycardie est classifiée comme étant d'origine ventriculaire - ce qui peut donc justifier l'application d'un choc de défibrillation. Dans le cas d'un défibrillateur double chambre, l'algorithme d'analyse morphologique VTC peut être amélioré en prenant en compte des critères supplémentaires, non morphologiques (V>A et stabilité).

Une autre méthode, dénommée "MD" (*Morphology Discrimination*) et décrite dans US 7 149 569 B1 (Pacesetter Inc.) utilise un algorithme qui cherche à calculer un pourcentage de correspondance entre un battement modèle et chaque battement de l'arythmie à analyser, ce pourcentage étant fonction de l'amplitude, de la polarité et de l'ordre des pics. Si au moins cinq battements parmi huit présentent un pourcentage de correspondance inférieur à une valeur de seuil, alors l'arythmie est caractérisée comme. étant une tachycardie d'origine ventriculaire. (le seuil peut être programmé avec des valeurs comprises entre 30 % et 95 %). Les études cliniques montrent toutefois qu'il faut programmer cet algorithme afin qu'il prenne également en compte des critères non morphologiques (accélération, stabilité) de manière à obtenir des résultats satisfaisants.

Le WO 00/69517 A1 (Medtronic Inc.) décrit une troisième méthode, dénommée *Wavelet Dynamic Discrimination,* consistant à comparer la morphologie d'un rythme de base et la morphologie, de la tachycardie en se basant sur la différence entre des coefficients d'ondelettes, cette différence étant exprimée en un pourcentage de correspondance. Les battements pour lesquels ce pourcentage est inférieur à une valeur nominale de 70 % sont classifiés comme étant d'origine ventriculaire, à la suite de quoi une tachycardie est classifiée comme étant d'origine ventriculaire si au moins six battements sur huit répondent à ce critère.

Une autre méthode encore est décrite par le US 2003/0083587 A qui prévoit, dans un dispositif double-chambre, de construire un vectocardiogramme (VCG) dont l'extrémité du vecteur décrit une boucle. La projection de cette boucle dans un plan donné permet d'évaluer la présence éventuelle de TV ou de TSV en combinant les variations de l'onde P (auriculaire) et celles de l'onde QRS (ventriculaire).

En tout état de cause, quelle que soit la technique mise en oeuvre, les algorithmes proposés jusqu'à présent continuent néanmoins à être leurrés dans certaines situations cliniques particulières, avec souvent pour conséquence un faux diagnostic de TV et donc le risque d'appliquer une thérapie inappropriée.

Le but de l'invention est de remédier aux inconvénients précités, en proposant une nouvelle technique d'analyse permettant d'éliminer quasiment tout risque de faux diagnostic de TV (faux positif ou faux négatif) lors de la discrimination entre TV et TSV, donc de réduire le nombre de chocs inappropriés dus à une mauvaise discrimination, et ceci dans toutes les situations cliniques susceptibles d'être rencontrées, assurant donc une plus grande fiabilité d'analyse des tachyarythmies.

En d'autres termes, le but de l'invention est d'améliorer la prise de décision des défibrillateurs implantables dans la discrimination entre TV et TSV, en gagnant en spécificité sans compromettre la sensibilité.

L'idée de base de l'invention réside dans la constatation de ce que des paramètres pertinents de discrimination entre TV et TSV peuvent être obtenus en analysant des signaux EGM provenant de la même cavité (le ventricule) et recueillis concurremment sur deux voies distinctes, ces signaux étant combinés entre eux sous forme de deux composantes respectives appliquées à une analyse bidimensionnelle - en faisant donc abstraction de la dimension temporelle.

Les deux voies EGM différentes sont par exemple celle du signal unipolaire (recueilli entre le boîtier et l'une des électrodes distale ou proximale), et celle du signal bipolaire (recueilli entre les deux électrodes distale et proximale).

On notera incidemment que l'analyse 'bidimensionnelle" ou "en deux dimensions" (2D) ne doit pas être entendue de manière en elle-même limitative, et que l'invention s'applique aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

L'invention propose, comme avec les méthodes existantes, d'effectuer la discrimination TV/TSV à partir d'une mesure de la ressemblance des signaux enregistrés pendant l'arythmie à ceux enregistrés en rythme sinusal.

En revanche, dans la présente invention, cette discrimination TV/TSV est opérée à partir de la "boucle cardiaque" ou "vectogramme", qui est la représentation de l'un de ces signaux par rapport à l'autre, dans un espace à deux dimensions. Cet espace est typiquement un espace "voie unipolaire (en ordonnée) vs. voie bipolaire (en abscisse)". Chaque battement (ou fraction significative de battement) est alors représenté par son vectogramme dans le plan ainsi défini. En cas d'arythmie, le battement est comparé à un vectogramme de référence correspondant, obtenu en rythme sinusal. L'algorithme estime la plus ou moins grande similitude avec ce vectogramme de référence et discrimine en conséquence le type d'arythmie, TV (faible similitude) ou TSV (forte similitude).

L'invention est définie par la revendication 1.

Les sous-revendications 2 à 13 visent une première forme de mise en oeuvre, préférentielle, de l'invention, où chaque vectogramme (rythme sinusal et arythmie) est caractérisé par son vecteur tangent et sa courbure en chaque point.

Les sous-revendications 14 à 29 visent une seconde forme de mise en oeuvre de l'invention, où le vectogramme est caractérisé par projection dans une base orthonormée définie à partir du rythme sinusal par une analyse en composantes principales.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 illustre les signaux d'électrogramme obtenus, respectivement sur les voies bipolaire ventriculaire et unipolaire ventriculaire, pour un patient présentant un rythme sinusal.

La Figure 2 est homologue de la Figure 1, lors d'un épisode de tachycardie supraventriculaire.

La Figure 3 illustre les boucles cardiaques obtenues en combinant les deux signaux des Figures 1 et 2 pour un même patient, en rythme sinusal et lors d'un épisode de tachycardie supraventriculaire.

La Figure 4 est homologue de la Figure 3, pour un même patient en rythme sinusal et lors d'un épisode de tachycardie ventriculaire.

La Figure 5 illustre, pour un premier mode de mise en oeuvre de l'invention, des signaux d'électrogramme typiquement recueillis sur les voies bipolaire ventriculaire et unipolaire ventriculaire simultanément enregistrées pour un patient donné.

La Figure 6 illustre le vectogramme obtenu en combinant les deux signaux de la Figure 5, pour huit battements successifs.

La Figure 7 est un organigramme illustrant les différentes étapes de l'algorithme d'estimation du battement de référence en rythme sinusal.

Les Figures 8 et 9 illustrent la manière dont est analysée la corrélation entre les battements, destinée à discriminer entre battements en rythme sinusal et extrasystoles ventriculaires.

La Figure 10 illustre deux paramètres de caractérisation d'un vectogramme en un point donné, à savoir la courbure et le vecteur tangent unitaire en ce point.

La Figure 11 est un organigramme illustrant les différentes étapes de l'algorithme de classification morphologique destiné à déterminer la nature, ventriculaire ou supraventriculaire, d'une tachycardie détectée chez un patient.

La Figure 12 illustre graphiquement les différents paramètres calculés par l'algorithme de caractérisation pour un même patient, respectivement en rythme sinusal et lors d'un épisode de tachycardie supraventriculaire, ainsi que la manière d'analyser ces paramètres pour en déduire la nature de cette tachycardie.

La Figure 13 est homologue de la Figure 12, pour un patient en rythme sinusal et lors d'un épisode de tachycardie ventriculaire.

La Figure 14 est un schéma synoptique montrant la manière dont l'analyse morphologique selon l'invention peut être combinée à une analyse rythmologique pour améliorer la spécificité d'un dispositif existant.

La Figure 15 illustre, pour un second mode de mise en oeuvre de l'invention, les boucles cardiaques obtenues, respectivement dans le cas d'un rythme sinusal et d'une tachycardie supraventriculaire, lorsque les composantes de ces boucles sont projetées dans la base définie par le rythme sinusal.

La Figure 16 est homologue de la Figure 15, pour un rythme sinusal et une tachycardie ventriculaire.

La Figure 17 illustre les variations des signaux correspondant à un rythme sinusal et à une tachycardie ventriculaire, lorsque ces signaux sont projetés sur l'axe principal et sur l'axe secondaire de la boucle cardiaque, ces axes ayant été déterminés par une analyse en composantes principales, selon le second mode de mise en oeuvre de l'invention.

La Figure 18 illustre une technique permettant, après analyse de corrélation, de discriminer entre tachycardies ventriculaires et tachycardies supraventriculaires à partir d'une distribution caractéristique des résultats de la corrélation, toujours selon le second mode de mise en oeuvre de l'invention.

On va maintenant décrire deux exemples de réalisation de l'invention, appliqués à un dispositif médical implantable actif permettant de surveiller en continu le rythme cardiaque et délivrer si nécessaire au coeur des impulsions électriques de stimulation, de resynchronisation et/ou de défibrillation en cas de trouble du rythme détecté par cet implant.

En ce qui concerne ses aspects logiciels, l'invention peut être mise en oeuvre par une programmation appropriée du logiciel de commande d'un dispositif connu, par exemple de type stimulateur cardiaque ou défibrillateur, comprenant des moyens d'acquisition d'un signal fourni par des sondes endocavitaires.

L'invention peut notamment être appliquée à des dispositifs implantables tels que les appareils de la gamme *Ovatio* commercialisés par ELA Médical, Montrouge, France. Il s'agit de dispositifs à microprocesseur programmable auxquels est possible de transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Comme cela a été indiqué plus haut, la technique d'analyse de l'invention consiste à opérer une discrimination entre tachycardies ventriculaires TV (ou VT) et tachycardies supraventriculaires TSV (ou SVT) à partir des signaux d'électrogramme EGM recueillis sur deux voies distinctes et analysés en deux dimensions.

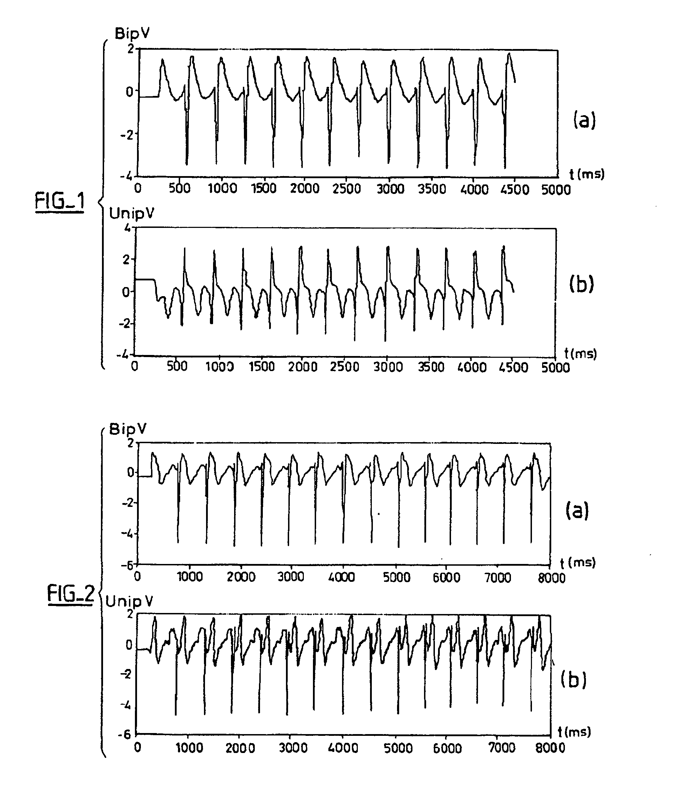

La Figure 1 illustre, dans le cas d'un patient présentant un rythme sinusal RS (ou SR) le tracé des électrogrammes BipV et UnipV, observés respectivement sur la voie bipolaire ventriculaire (Fig. 1a) et sur la voie unipolaire ventriculaire (Fig. 1b).

La Figure 2 illustre, de la même façon, les signaux correspondants BipV et UnipV observés dans le cas d'un patient présentant une TSV.

Ces signaux font l'objet d'un prétraitement approprié de filtrage, normalisation et centrage (ce prétraitement, classique en lui-même, ne faisant pas partie de l'invention).

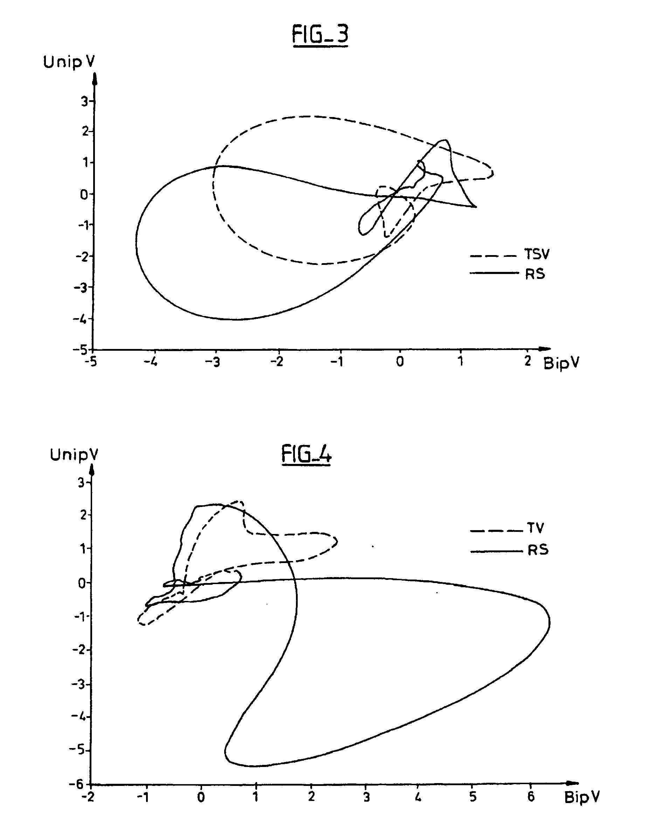

L'étape suivante consiste, une fois ces signaux recueillis (dans le domaine temporel), à tracer l'un des signaux en fonction de l'autre. La caractéristique obtenue, dénommée "boucle cardiaque", est illustré Figure 3, d'une part dans le cas d'un rythme sinusal (boucle en trait continu) et dans le cas d'un épisode de TSV (boucle en tiretés), chez le même patient. Chacune de ces boucles est représentative d'un battement cardiaque complet, soit en rythme sinusal, soit en TSV.

On verra toutefois plus bas qu'il n'est pas indispensable d'analyser la totalité du battement, mais que l'analyse d'une fraction significative de ce battement (typiquement, celle centrée autour du complexe QRS) est en général suffisante pour opérer la discrimination recherchée.

En comparant les deux boucles cardiaques illustrées Figure 3, on peut remarquer que la boucle RS (correspondant à un battement en rythme sinusal) et la boucle TSV (correspondant à un battement en tachycardie supraventriculaire) présentent une ressemblance du point de vue direction de la boucle, orientation de la boucle, sens de propagation ainsi que du point de vue de leur forme et de l'aire circonscrite.

En revanche, sur la Figure 4 qui illustre les boucles obtenues dans le cas d'un patient présentant un rythme sinusal (boucle en trait continu) avec des épisodes de TV (boucle en tiretés), la boucle obtenue en TV diffère considérablement de celle obtenue en RS, et l'on ne peut noter aucune similitude ni ressemblance.

L'invention propose, essentiellement, de systématiser cette approche par analyse des caractéristiques du vectogramme 2D, par rapport à un vectogramme de référence correspondant, obtenu en rythme sinusal.

*Premier mode de mise en oeuvre du procédé de l'invention*

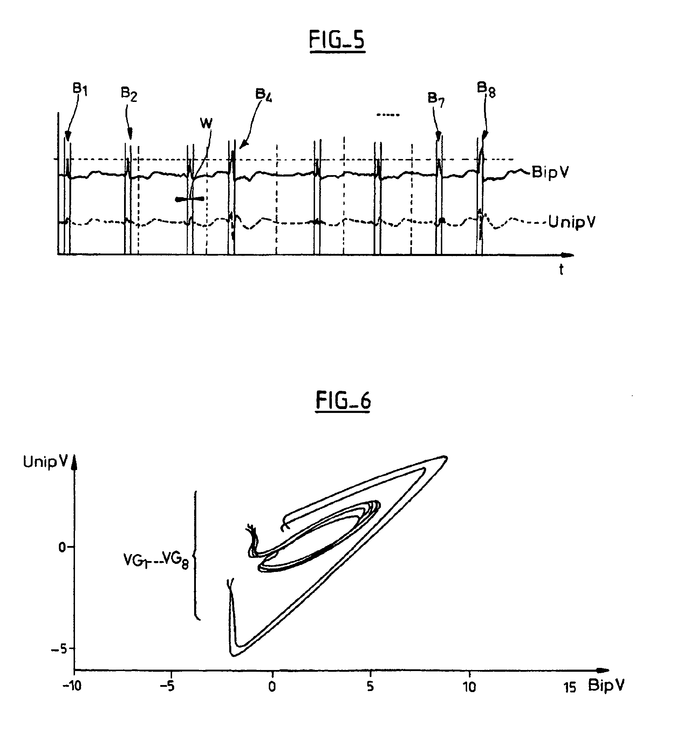

On va maintenant décrire un premier mode de mise en oeuvre de l'invention, en référence aux Figures 5 à 14.

Dans cette approche, après chaque détection d'un pic de dépolarisation du signal bipolaire BipV (correspondant à une onde R détectée), le battement correspondant est isolé par une fenêtre fixe W de quelques dizaines de millisecondes centrée sur ce pic de dépolarisation, par exemple une fenêtre de largeur W = 80 ms correspondant à 80 points pour une fréquence d'échantillonnage de 1000 Hz. Cette valeur typique de 80 ms permet de bien isoler le complexe QRS pour en analyser la morphologie, sans trop inclure de bruit autour, bruit correspondant à la ligne de base après la fin du QRS.

Le dispositif garde en mémoire une pluralité de battements successifs, par exemple les huit derniers battements $B_1$ à $B_8$, comme illustré Figure 5, ces battements étant enregistrés simultanément sur la voie bipolaire ventriculaire (BipV) et la voie unipolaire ventriculaire (UnipV).

La fraction de chacun de ces battements comprise à l'intérieur de la fenêtre W est alors représentée sous forme de vectogramme, considéré dans le plan constitué de la voie bipolaire en abscisse et de la voie unipolaire en ordonnée. On notera que le vectogramme correspondant à chacun des battements n'est pas une boucle fermée, dans la mesure où il ne correspond qu'à une partie de la boucle cardiaque complète, à savoir le complexe QRS isolé à l'intérieur de la fenêtre W.

L'analyse nécessite la création préalable d'un battement de référence, moyenné à partir d'une succession de battements en rythme sinusal.

Il est toutefois nécessaire, même en l'absence de tachycardie, d'exclure certains battements non significatifs : sur les vectogrammes tracés Figure 6, on voit ainsi que parmi les huit vectogrammes $VG_1$ ... $VG_8$, deux d'entre eux s'écartent notablement de la forme des autres : il s'agit de vectogrammes correspondant à des extrasystoles ventriculaires ou ESV (battements $B_4$ et $B_8$ sur la Figure 5), qu'il est nécessaire de repérer et d'exclure du calcul du battement de référence moyen, car leur morphologie n'est en aucune façon représentative.

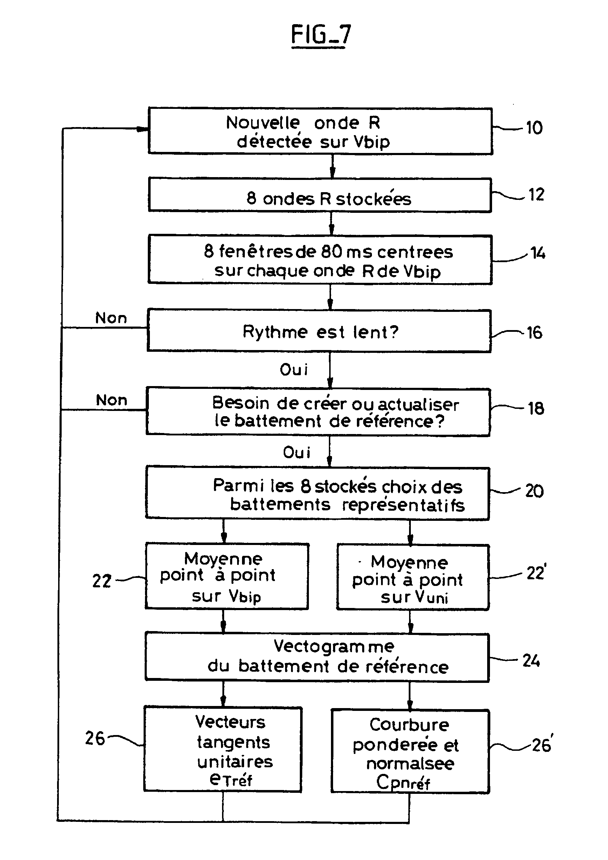

Sur la Figure 7, on a illustré un algorithme d'analyse du rythme sinusal et de détermination d'un battement de référence représentatif.

A chaque détection d'une onde R sur la voie bipolaire (étape 10), le dispositif stocke les huit ondes successives (étape 12) et isole le complexe QRS dans la fenêtre W pour chacun des battements recueillis sur la voie bipolaire (étape 14).

En l'absence de tachycardie (test 16), l'algorithme détermine s'il y a ou non besoin de créer ou actualiser le battement de référence (test 18). En effet, même s'il existe déjà un battement de référence, il peut être souhaitable de l'actualiser à intervalles réguliers (typiquement au moins une fois par jour, ou au moins une fois par heure après l'implantation de manière à prendre en compte le phénomène de maturation des électrodes après l'implantation de la sonde), et/ou en fonction de l'état du patient (repos/exercice, etc.).

Dans l'affirmative, l'algorithme sélectionne les battements représentatifs parmi les huit battements mémorisés, en isolant et en éliminant les ESV et les artefacts divers tels que fenêtres mal centrées, etc.

Une première méthode, simple, de sélection des battements représentatifs consiste à ne conserver que les complexes pour lesquels les intervalles RR sont stables, et moyenner point à point les complexes répondant à ces critères.

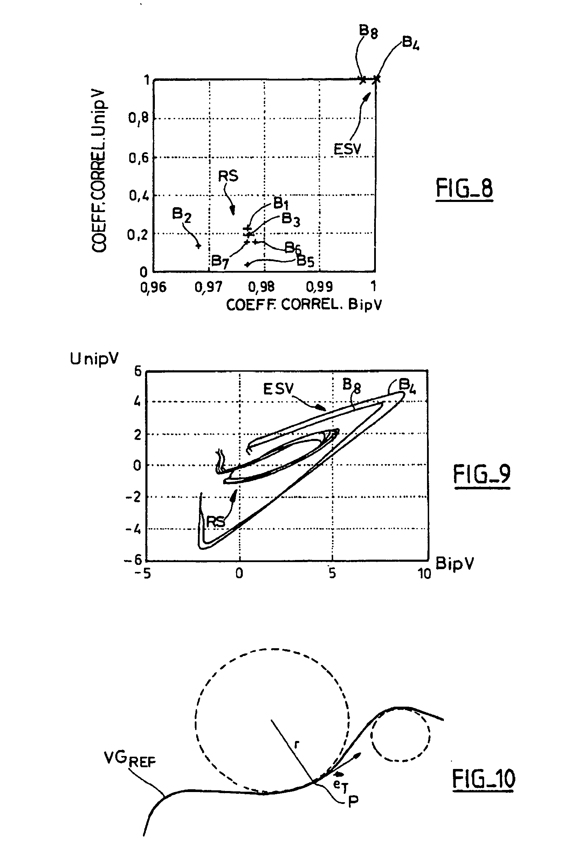

Une autre méthode, illustrée en référence aux Figures 8 et 9, consiste à analyser la morphologie des huit battements par corrélation croisée.

À cet effet, un battement est pris au hasard comme référence, par exemple le quatrième des huit battements de la Figure 5 (qui est en l'espèce une ESV). Un coefficient de corrélation est calculé entre ce battement de référence et chacun des sept autres battements, et ceci à la fois pour le signal bipolaire et pour le signal unipolaire. Les coefficients de corrélation correspondants $C_{BipV}(i,4)$ et $C_{UnipV}(i,4)$ de chaque battement $B_i$ peuvent être alors représentés dans un plan par un point d'abscisse $C_{BipV}(i,4)$ et d'ordonnée $C_{UnipV}(i,4)$, le point correspondant au quatrième battement ($i=4$) étant le point $(1,1)$.

Si tous les coefficients de corrélation sont supérieurs à 0,9, alors le battement de référence en rythme lent est calculé en moyennant point à point les huit battements, ceci pour chacune des deux voies bipolaire et unipolaire (étapes 22 et 22' sur la Figure 7).

En revanche, s'il existe des valeurs inférieures à 0,9 (comme dans le cas de l'exemple illustré), alors un algorithme itératif de "*clustering* supervisé" est appliqué à ces huit points, par exemple de type "algorithme des K-moyennes". Un tel algorithme, en lui-même connu, consiste à partitionner les données en K classes homogènes (ici K=2, principalement pour séparer les cycles normaux des ESV) en minimisant la variance intra-classe de manière à obtenir, de manière itérative, des "*clusters*" (agrégats) basés sur la distance euclidienne entre les points. Pour chaque point, si sa distance au centre du *cluster* est supérieure à la moitié de la distance entre les deux centres des *clusters* respectifs, alors on considérera que ce point n'appartient à aucun *cluster* (ce n'est pas le cas dans l'exemple illustré Figure 9, où la distance intra-*cluster* est notablement inférieure à la moitié de la distance inter-*cluster*) - cette condition est nécessaire pour isoler tout artéfact, par exemple dû à un mauvais centrage de la fenêtre W. Enfin, l'algorithme choisit le *cluster* comportant le plus grand nombre d'éléments, à savoir le *cluster* en bas à gauche sur la Figure 8.

Le battement de référence en rythme lent est calculé sur chacune des deux voies bipolaire et unipolaire (étapes 22 et 22') en moyennant point à point les battements correspondant au *cluster* choisi : dans l'exemple, ce seront les vectogrammes référencés "RS" de la Figure 9, correspondant au *cluster* "RS" de la Figure 8, qui seront moyennés point à point pour obtenir le battement de référence, tandis que les deux vectogrammes référencés "ESV" sur la Figure 9, correspondant aux deux battements $B_4$ et $B_8$ du *cluster* "ESV" de la Figure 8, seront éliminés car correspondant à des ESV (ou à des artefacts).

À partir des moyennes point à point des battements sur les voies bipolaire et unipolaire, l'algorithme détermine alors un vectogramme du battement de référence (étape 24 de la Figure 7), en portant en abscisse les variations de la voie bipolaire et en ordonnée celles de la voie unipolaire, pour chacun des points d'échantillonnage des signaux à l'intérieur de la fenêtre W.

Ce vectogramme est ensuite caractérisé en chacun de ses points. L'invention propose, par exemple, de réaliser cette caractérisation pardeux descripteurs (Figure 10) : le vecteur tangent unitaire $\vec{e}_T$ et la courbure $c$ (inverse du rayon de courbure r) au point P du vectogramme de référence $VG_{REF}$, et ceci pour les différents points échantillonnés successifs du vectogramme (étapes 26 et 26' de la Figure 7).

Un autre descripteur possible est la norme du vecteur tangent.

Le vecteur tangent unitaire $\vec{e}_T$ en un point donné peut être déterminé par une technique en elle-même connue, notamment avec un filtre discret qui approxime les dérivées premières, par exemple sur quatre points pour une fréquence d'échantillonnage de 1000 Hz, ce filtrage étant suivi d'une normalisation (pour que le vecteur tangent soit unitaire).

Quant à la courbure $c$, elle peut être calculée en un point donné du vectogramme à partir des dérivées premières et des dérivées secondes, calculées de façon analogue aux dérivées premières. Avantageusement, pour donner plus d'importance aux zones d'intérêt du vectogramme où les points sont les plus espacés, la courbure est ensuite pondérée par une puissance de la distance entre les points. Cette distance est calculée à partir d'un filtre discret appliqué sur les distances euclidiennes entre deux points successifs de l'espace du vectogramme. Finalement la courbure est normalisée.

Le vectogramme de référence a été ainsi déterminé, et caractérisé par son vecteur tangent et sa courbure en chaque point.

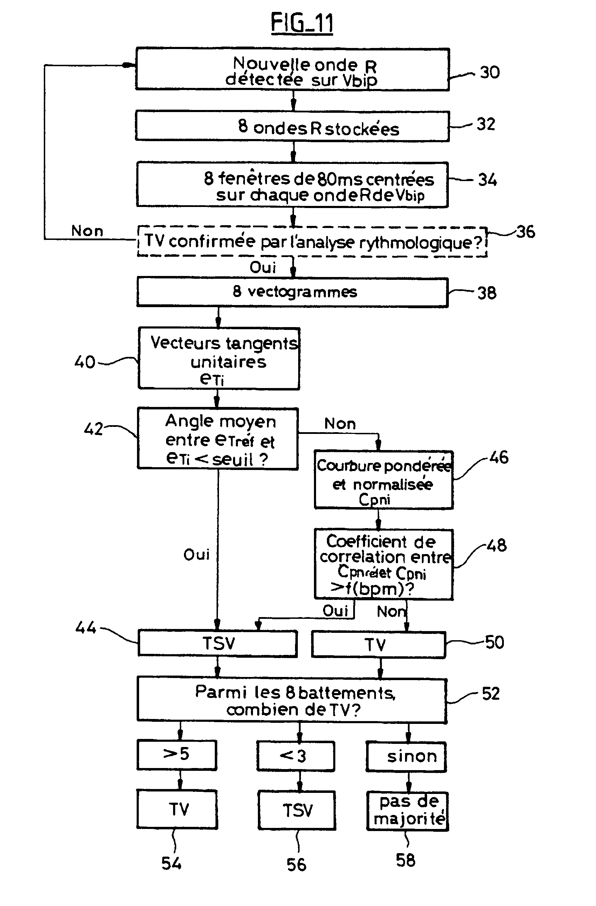

En cas de tachycardie, le dispositif sera alors en mesure de déterminer la nature de cette tachycardie par une analyse morphologique impliquant une comparaison avec le vectogramme de référence ainsi défini. L'algorithme général de classification des tachycardies est illustré Figure 11.

Le dispositif détecte et mémorise les huit derniers battements, en ne conservant que les informations centrées sur une fenêtre W autour du pic de dépolarisation du signal bipolaire (étapes 30, 32 et 34). La manière de procéder est la même que celle exposée plus haut en référence à la Figure 5, les étapes 30, 32 et 34 étant semblables aux étapes 10, 12 et 14 précédemment explicitées.

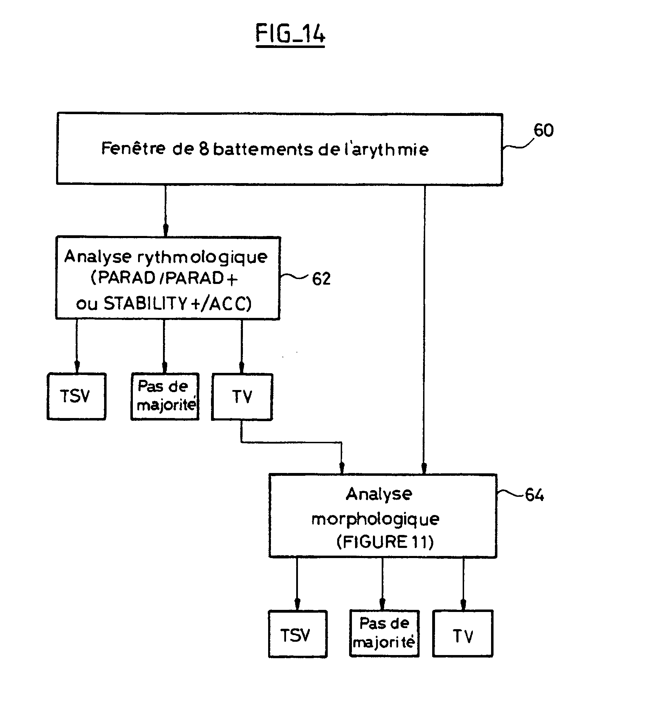

L'algorithme peut éventuellement conditionner la poursuite de l'analyse morphologique à l'existence d'une TV confirmée préalablement par l'analyse rythmologique (test 36) ; par exemple, par des algorithmes tels que PARAD, PARAD+ ou STABILITY+ mis en oeuvre dans les dispositifs ELA Medical et décrits dans les documents précités EP 0 626 182 A1 (ELA Medical) et autres. La combinaison de l'analyse rythmologique et de l'analyse morphologique sera décrite plus loin en référence à la Figure 14, mais on peut déjà noter que le conditionnement de l'analyse morphologique à la détection préalable d'une TV par l'analyse rythmologique n'est pas une caractéristique nécessaire de la mise en oeuvre de l'invention, et que l'étape 36 est donc une étape facultative.

L'étape suivante (étape 38) consiste à tracer les vectogrammes des huit derniers battements, et de les caractériser en chacun de leurs points par les deux descripteurs (vecteur tangent unitaire et courbure pondérée et normalisée).

La comparaison d'un vectogramme obtenu en tachycardie avec le vectogramme de référence obtenu en rythme sinusal pour le même patient se fait par le calcul de deux quantités :

- l'angle moyen a entre les vecteurs tangents unitaires des deux vectogrammes respectifs, et
- le coefficient de corrélation $c$ entre les courbures des deux vectogrammes respectifs.

La discrimination entre TV et TSV sera opérée sur les valeurs de a et de $c$ par exemple par comparaison avec des seuils de décision déterminés au préalable à partir d'une base d'apprentissage. Ainsi :

- si l'angle moyen a est inférieur à une valeur donnée (étapes 40 et 42), ou si le coefficient de corrélation $c$ est supérieur à un seuil donné dépendant de la fréquence cardiaque (étapes 46 et 48), alors le battement correspondant de l'arythmie est classé comme étant d'origine supraventriculaire (étape 44) ;
- sinon il est classé comme étant d'origine ventriculaire (étape 50).

En ce qui concerne le seuil pour le coefficient de corrélation, celui-ci correspond à une fonction quadratique de la fréquence cardiaque, cette fonction étant calculée sur l'ensemble d'apprentissage par des méthodes classiques de classification supervisée, comme la méthode des moindres carrés. La classification supervisée consiste à inférer à partir d'un échantillon de données classées une frontière de décision qui sépare les deux classes en minimisant l'erreur quadratique (au sens des moindres carrés) entre les valeurs vraies (par exemple 1 pour TV et -1 pour TSV) et les valeurs prédites par le classifieur.

L'étape suivante (étape 52) consiste à comparer les résultats obtenus pour chacun des huit battements successifs :

- si au moins six des huit battements sont classés comme étant d'origine ventriculaire, alors l'arythmie à ce stade est classée comme étant d'origine ventriculaire d'après l'analyse morphologique (étape 54) ;

- si au moins six des huit derniers battements sont classés comme étant d'origine supraventriculaire, l'arythmie à ce stade est classée comme étant d'origine supraventriculaire d'après l'analyse morphologique (étape 56) ;
- sinon, l'arythmie n'est pas classée, dans la mesure où l'analyse morphologique ne dégage aucune tendance majoritaire significative (étape 58).

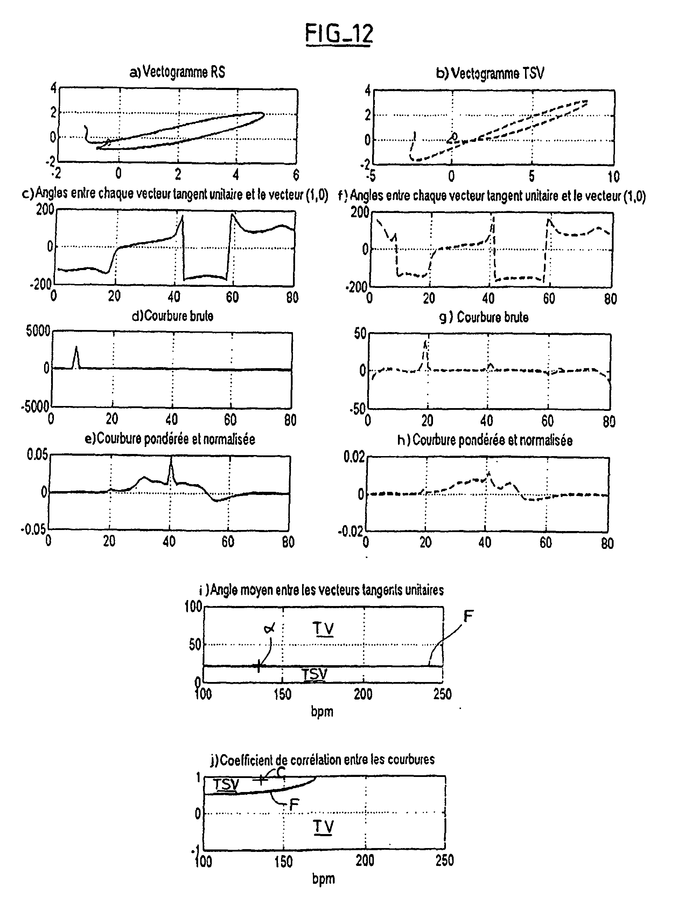

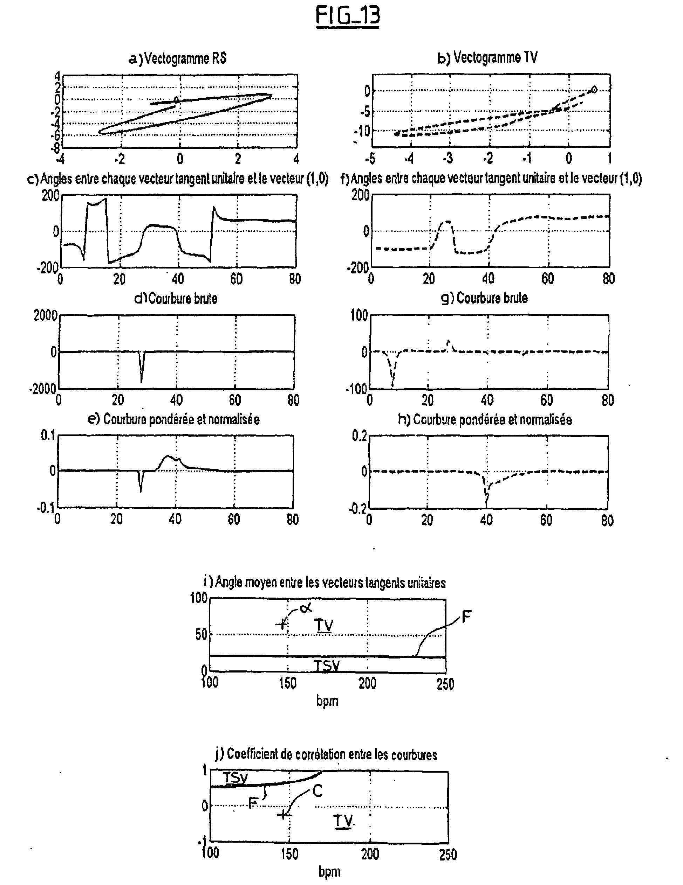

Les Figures 12 et 13 montrent deux exemples de classification obtenues par application de la méthode de l'invention, respectivement pour un premier patient en rythme sinusal et lors d'un épisode de TSV, et pour un second patient en rythme sinusal et lors d'un épisode de TV :

- les Figures 12a et 13a montrent les vectogrammes correspondant à deux battements de référence, calculés comme précédemment en rythme sinusal pour les deux patients respectifs ;
- la Figure 12b illustre le vectogramme en TSV du premier patient, et la Figure 13b illustre le vectogramme en TV du second patient.
- les Figures 12c, 12d et 12e montrent respectivement, pour le vectogramme du battement de référence du premier patient (vectogramme de la Figure 12a) : les variations de l'angle moyen entre le vecteur tangent unitaire et l'axe des abscisses ; la courbure brute ; et la courbure pondérée et normalisée;
- les Figures 13c, 13d et 13e sont homologues des précédentes, pour le vectogramme du battement de référence du second patient (vectogramme de la Figure 13a) ;
- les Figures 12f, 12g et 12h sont homologues des précédentes, pour le vectogramme du premier patient obtenu en épisode d'arythmie supraventriculaire (vectogramme de la Figure 12b) ;
- les Figures 13f, 13g et 13h sont homologues des précédentes, pour le vectogramme du second patient obtenu en épisode d'arythmie ventriculaire (vectogramme de la Figure 13b) ;
- les Figures 12i et 13i indiquent l'angle moyen a entre les vecteurs tangents unitaires des vectogrammes en arythmie et en rythme sinusal, par rapport à la frontière de décision F entre TV et TSV ;
- les Figures 12j et 13j indiquent le coefficient de corrélation $c$ entre les courbures des vectogrammes en arythmie et en rythme sinusal, par rapport à la frontière de décision F entre TV et TSV.

Dans le cas du premier patient (Figure 12), l'analyse des vecteurs tangents unitaires n'est pas suffisante pour conclure qu'il s'agit d'une TSV (sur la Figure 12i, le point a est trop proche de la frontière F), mais l'étude de la courbure confirme bien la TSV (Figure 12j).

Pour le deuxième patient, les deux critères montrent clairement qu'il s'agit d'une TV (Figure 13i et Figure 13j).

La Figure 14 est un schéma synoptique montrant la manière dont il est possible de combiner analyse rythmologique (selon des techniques connues) et analyse morphologique (selon l'invention) pour permettre au dispositif de prendre une décision globale sur la classification de l'arythmie, et donc sur l'opportunité d'appliquer ou non au patient un choc de défibrillation.

Pour un défibrillateur double chambre, l'analyse morphologique est notamment utile lorsque l'association atrio-ventriculaire est de 1:1, car dans ce cas l'accélération est soudaine et l'origine de cette accélération n'est pas évidente (tachycardie auriculaire/tachycardie ventriculaire). Ou encore lorsque les intervalles RR sont stables et qu'il n'y a pas d'association atrio-ventriculaire (fibrillation auriculaire/tachycardie ventriculaire), car l'analyse rythmologique est souvent insuffisante pour déterminer à coup sûr l'origine de l'arythmie.

Pour un défibrillateur simple chambre, l'analyse morphologique permet d'éviter des chocs inappropriés. En effet, la conjonction d'une situation avec intervalles RR stables, accélération soudaine et absence de cycle long, considérée par l'analyse rythmologique comme nécessitant une thérapie, peut très bien caractériser dans certaines situations une tachycardie supraventriculaire, qui ne justifie pas une telle thérapie. L'analyse morphologique selon l'invention permettra, précisément, de discriminer une telle situation.

En référence à la Figure 14, à partir de huit battements successifs recueillis en arythmie (étape 60), le dispositif opère concurremment une analyse rythmologique (étape 62) et une analyse morphologique (étape 64 de la manière explicitée plus haut sur la Figure 11). L'analyse rythmologique opère la classification entre TV, TSV ou arythmie non significative (pas de majorité sur les huit battements) et l'analyse morphologique fait de même. Avantageusement, l'analyse morphologique n'est exécutée, ou prise en compte, que si l'analyse rythmologique conclut que l'arythmie est d'origine ventriculaire (TV). Dans ce cas, le but recherché par l'analyse morphologique est d'éviter un choc inapproprié, en prenant comme hypothèse que la sensibilité de l'analyse rythmologique est bien égale à l'unité :

- si l'analyse rythmologique conclut que la tachyarythmie est d'origine supraventriculaire (TSV), ou indéterminée (absence de majorité), alors aucune thérapie ne sera déclenchée, quel que soit le résultat de l'analyse morphologique ;
- si, en revanche, l'analyse rythmologique conclut que l'arythmie est d'origine ventriculaire (TV) et qu'elle est persistante, alors la thérapie ne sera déclenchée que si l'analyse morphologique, quant à elle, confirme l'origine ventriculaire de cette arythmie, à la fois lorsque cette dernière est détectée et pendant la persistance (douze cycles dans la zone des TV).

*Deuxième mode de mise en oeuvre du procédé de l'invention*

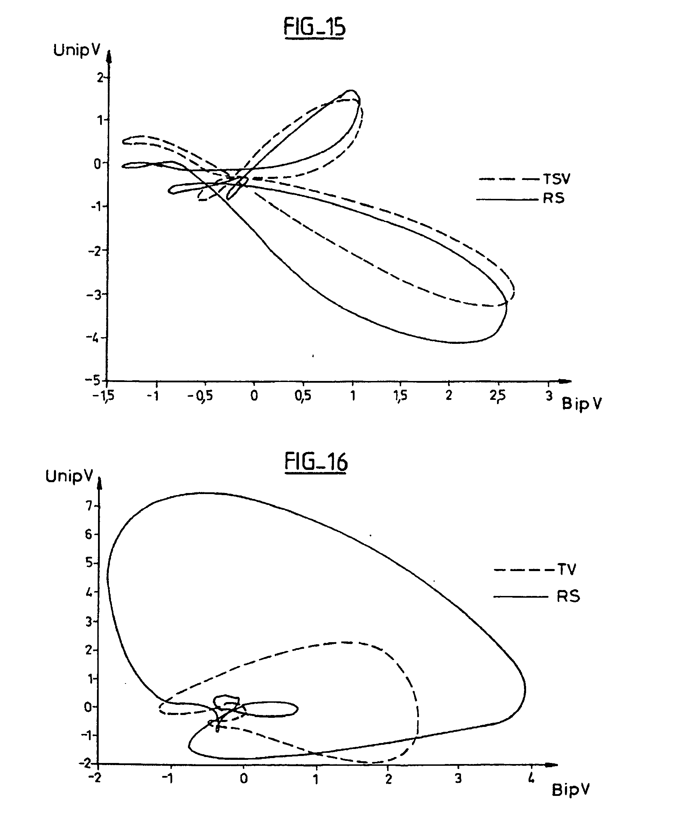

Les Figures 15 à 18 illustrent un autre mode de mise en oeuvre de l'invention.

Ce deuxième mode de mise en oeuvre est également basé sur l'analyse et la caractérisation du vectogramme, mais à partir d'autres critères que ceux décrits plus haut à propos du premier mode de mise en oeuvre (vecteur tangent unitaire et courbure pondérée et normalisée en chaque point). Les considérations relatives à la manière dont il est possible de combiner analyse rythmologique et analyse morphologique, expliquée notamment en relation avec la Figure 14, restent toutefois parfaitement applicables à ce deuxième mode de mise en oeuvre.

Dans ce deuxième mode de mise en oeuvre, la base orthonormée dans laquelle sera représenté le vectogramme UnipV = f(BipV) est définie par une analyse en composantes principales (analyse dite ACP) à partir du rythme sinusal.

Cette analyse ACP, qui est une technique en elle-même connue, peut être effectuée pour chaque battement, et elle permet de déduire l'axe électrique du coeur, qui est un indicateur de la direction générale que prend l'onde électrique lorsqu'elle se propage dans les ventricules : la voie de plus grande dynamique est celle qui présente la projection la plus grande, la direction correspondante étant dénommée "axe principal" ; cet axe peut être complété par deux autres axes dits "axes secondaires, perpendiculaires entre eux et à l'axe principal.

Dans le cas présent, on n'effectuera l'analyse qu'en deux dimensions (en ne considérant donc qu'un seul des axes secondaires).

En effet, comme on le verra, la technique de l'invention permet de discriminer entre TV et TSV à partir de deux électrodes seulement, ce qui permet une implémentation de cette technique dans un défibrillateur simple chambre.

Toutefois, bien que l'analyse d'une caractéristique 2D soit suffisante pour atteindre le but recherché, dans une mise en oeuvre perfectionnée l'analyse peut être effectuée sur la base d'une caractéristique 3D, obtenue à partir de trois électrodes.

On va maintenant décrire le principe de l'analyse en composantes principales ACP permettant de définir le repère de référence orthonormé.

Soient $S1$ et $S2$ les deux signaux sur les voies respectives $A$ (BipV) et $B$ (UnipV) représentant un battement cardiaque moyenné par exemple sur quinze battements sinusaux successifs. Chaque signal est constitué de $N$ points représentés dans la base des électrodes ($A$, $B$), les coordonnées du $i$ème point étant ($S1(i)$, $S_2(i)$).

Pour l'analyse en composantes principales, on fait l'approximation que ces N points forment une ellipse, ce qui permet de calculer :

- les axes de cette ellipse qui forment la base ACP,
- la longueur de chacun d'eux.

Ces deux valeurs permettent d'une part d'identifier la direction principale de l'ellipse (donc la direction d'étalement du vectogramme) et d'autre part de quantifier sa dimension et son aire d'autre part.

On recherche donc les coordonnées de ces N points dans la base ACP ($P_1$, $P_2$), ce qui nécessite le calcul de la matrice de passage de la base ($A$, $B$) à la base ($P_1$, $P_2$).

La matrice de passage s'obtient par diagonalisation de la matrice de covariance C associée aux $N$ points. Calculer la matrice de covariance revient à faire l'approximation que les $N$ points font partie d'une ellipse. En diagonalisant cette matrice, on obtient :

- les axes de cette ellipse, définis par les vecteurs propres de $C$, et
- la longueur de chacun de ces axes, indiquée par la valeur propre cor respondante.

Le vecteur propre qui a la plus grande valeur propre donne ainsi la direction de la plus grande dispersion de nuage de points.

On calcule ensuite les valeurs propres ($\lambda_i$) $i=1,2$ et les vecteurs propres ($V1, V2$) associés à la matrice $C$.

On cherche la matrice $D$ telle que :

$$D = P^{-1} . C . P$$

Où $D$ est la matrice diagonale des valeurs propres :

$$D = \begin{bmatrix} \lambda_1 & 0 \\ 0 & \lambda_2 \end{bmatrix}$$

et $P$ la matrice de passage de la base ($P_1$, $P_2$) à la base ($A$, $B$) constituée des vecteurs propres de $C$. Ainsi, l'inverse de $P$ s'écrit :

$$P^{-1} = \begin{bmatrix} P_1 & P_2 \end{bmatrix}$$

où $P_i$ est le vecteur colonne $i$ de la base ACP (c'est-à-dire le vecteur propre associé à la valeur propre $\lambda_i$) exprimé dans la base ($A$, $B$). En classant les $\lambda_i$ par ordre décroissant, le vecteur $P_1$ représente la direction de plus grand étalement du nuage, et le vecteur $P_2$ la deuxième.

Le signal ($S_1^{ACP}$, $S_2^{ACP}$) dans cette nouvelle base ($P_1$, $P_2$) s'écrit donc :

$$[S_1^{ACP}, S_2^{ACP}] = P^{-1} \cdot \begin{bmatrix} S_1 \\ S_2 \end{bmatrix}$$

Comme indiqué plus haut, selon l'invention la base ACP est calculée sur la base du rythme sinusal, avant de projeter les données du rythme sinusal et les données de la tachycardie sur cette même base.

La Figure 15 illustre le résultat obtenu après ce changement de base, pour un patient présentant un rythme sinusal RS (boucle en trait continu) et des épisodes de tachycardie supraventriculaire TSV (boucle en tiretés).

La Figure 16 est homologue de la Figure 15, pour un patient présentant un rythme sinusal RS (boucle en trait continu) et des épisodes de tachycardie ventriculaire TV (boucle en tiretés).

En comparant les Figures 5 et 6, on observe une ressemblance très étroite entre les boucles RS et TSV du point de vue direction, sens, forme, surface et morphologie, tandis qu'aucune ressemblance significative ne peut être observée entre les boucles RS et TV.

L'étape suivante de l'analyse consiste à déterminer un certain nombre de paramètres descripteurs de la morphologie de ces boucles de manière à pouvoir opérer, dans les meilleures conditions, une discrimination entre TV et TSV pour un patient présentant des épisodes de tachycardie. L'analyse en composantes principales effectuée à l'étape précédente permet notamment d'extraire les paramètres descripteurs suivants (la manière d'obtenir ces paramètres sera décrite plus bas) :

- l'axe principal, qui est le vecteur propre de la matrice de covariance associé à la plus grande valeur propre ;
- l'axe secondaire, qui est le vecteur propre de la matrice de covariance associé à la deuxième valeur propre ;
- les dimensions de ces axes, qui sont les deux valeurs propres de la matrice de covariance ;
- les angles que font les deux axes avec l'axe OX, à partir des calculs des sinus et des cosinus.

Pour pouvoir extraire à partir de l'ACP des paramètres mathématiques descripteurs de la morphologie des boucles, chaque signal (rythme sinusal et tachycardie) est ensuite projeté sur sa propre base, de manière à pouvoir observer le signal unidimensionnel correspondant (qui est donc un signal dans le domaine temporel), puis comparer les formes afin d'extraire les paramètres morphologiques qui différencient les SVT des VT.

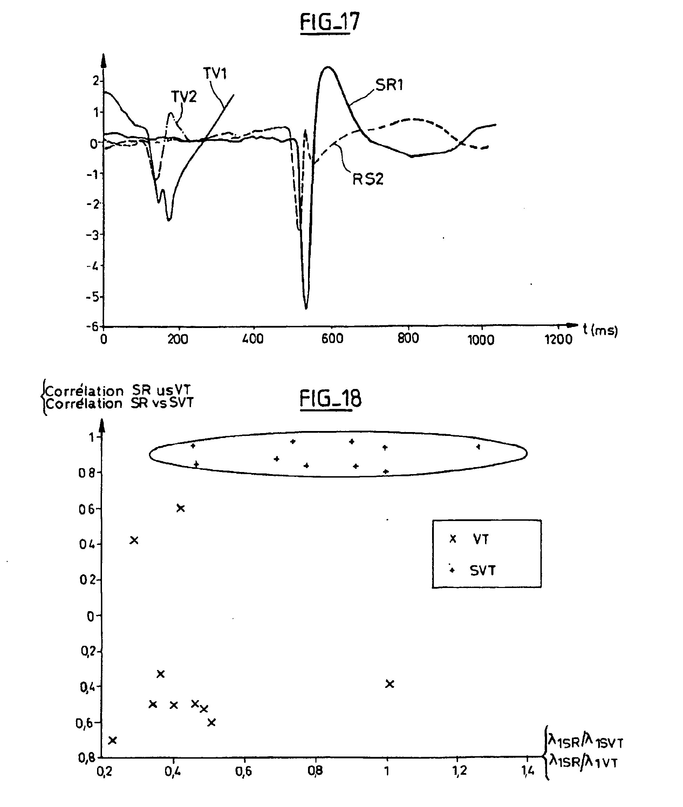

La Figure 17 illustre ces signaux unidimensionnels :

- les tracés référencés RS1 et TV1 représentent les composantes ACP projetées sur l'axe principal du repère de référence, respectivement pour un battement en rythme sinusal et pour un battement en tachycardie ventriculaire ;
- les tracés référencés RS2 et TV2 correspondent aux mêmes composantes ACP respectives, projetées sur l'axe secondaire du repère de référence.

Une fois cette étape réalisée, il est possible d'extraire des paramètres représentatifs tels que :

- hauteur maximale des signaux (sur les deux axes, principal et secondaire) ;
- hauteur minimale des signaux (sur les deux axes, principal et secondaire) ; et/ou
- largeur des signaux (sur les deux axes, principal et secondaire).

À partir de ces paramètres morphologiques, l'algorithme calcule ensuite des coefficients de corrélation entre, d'une part, les signaux RS et TSV et, d'autre part, les signaux RS et TV, ces coefficients étant calculés sur la voie principale et la voie secondaire. L'erreur quadratique moyenne par rapport au rythme sinusal est également calculée, pour les battements TSV et pour les battements TV.

La distribution obtenue dans l'un et l'autre cas de tachycardie est illustrée notamment Figure 18, où l'on a porté :

- en abscisse, le rapport des valeurs propres sur la voie principale du rythme sinusal et de la TSV ou de la TV, et
- en ordonnée, le coefficient de corrélation entre RS et TSV, ou entre RS et TV.

Cette distribution montre que les données obtenues dans le cas d'une TV et dans le cas d'une TSV sont très bien séparées et qu'il est ainsi possible d'opérer une classification des tachycardies et une discrimination pertinente par mise en oeuvre par exemple d'un classifieur linéaire ou d'un classifieur neuronal, de la manière qui sera décrite plus bas.

On va maintenant décrire plus en détail les paramètres descripteurs de la morphologie des boucles 2D pouvant servir à opérer cette classification des tachycardies.

À partir de l'EGM en rythme sinusal du patient :

- première valeur propre $\lambda_{1,SR}$ et seconde valeur propre $\lambda_{2,SR}$ du calcul d'analyse en composantes principales ;
- angle $\theta_{SR}$ entre le premier axe principal du battement et la première voie d'enregistrement ;
- rapport $R_{1,SR}$ entre l'amplitude maximale et l'amplitude minimale du complexe de dépolarisation sur la première voie principale ; et
- rapport $R_{2,SR}$ entre l'amplitude maximale et l'amplitude minimale du complexe de dépolarisation sur la deuxième voie.

De la même manière, pour les battements en tachycardie (TV ou TSV) il est possible d'obtenir les paramètres suivants :

- première valeur propre $\lambda_{1,TR}$ et seconde valeur propre $\lambda_{2,TR}$ du calcul d'analyse en composantes principales ;
- angle $\theta_{TR}$ entre le premier axe principal du battement et la première voie d'enregistrement ;
- rapport $R_{1,TR}$ entre l'amplitude maximale et l'amplitude minimale du complexe de dépolarisation sur la première voie principale ; et
- rapport $R_{2,TR}$ entre l'amplitude maximale et l'amplitude minimale du complexe de dépolarisation sur la deuxième voie.

Pour la comparaison du battement sinusal et du battement en tachycardie les paramètres représentatifs suivants peuvent être utilisés :

- maximum de corrélation $M_1$ entre le tracé sur la première voie principale du battement sinusal et du battement en tachycardie ;
- maximum de corrélation $M_2$ entre les tracés sur la seconde voie principale ; et/ou
- erreur quadratique moyenne $EQM$ entre les deux battements sur la première voie principale.

À partir de ces paramètres, il est possible de calculer diverses expressions représentatives, désignées ci-après $D_1$ à $D_5$.

L'expression $D_1$ ci-dessous, qui est le rapport de la première et de la seconde valeur propre du calcul en composantes principales, traduit la forme de la boucle du vectogramme associée au battement, donc le rapport de forme entre le battement sinusal et le battement en tachycardie :

$$D_1 = \frac{(\lambda_1/\lambda_2)_{SR}}{(\lambda_1/\lambda_2)_{TR}}$$

L'expression $D_2$ ci-dessous traduit le rapport entre la fraction d'information contenue sur la voie principale pour le battement sinusal et celle contenue sur la voie principale pour le battement en tachycardie ($\lambda_1/(\lambda_1+\lambda_2)$) reflétant la

proportion d'information exprimée par la voie principale par rapport à l'information totale disponible sur les deux voies) :

$$D_2 = \frac{(\lambda_1/(\lambda_1 + \lambda_2))_{SR}}{(\lambda_1/(\lambda_1 + \lambda_2))_{TR}}$$

Si l'on désigne θ l'angle formé par l'axe principal avec la première voie d'enregistrement, l'expression $D_3$ ci-dessous traduit le rapport des directions de propagation du battement en rythme sinusal et en tachycardie :

$$D_3 = \frac{\theta_{SR}}{\theta_{TR}}$$

Enfin, les expressions $D_4$ et $D_5$ ci-dessous traduisent les dissemblances des tracés sur la première voie principale et la seconde voie principale des deux battements, sinusal et en tachycardie :

$$D_4 = \frac{R_{1,SR}}{R_{1,TR}}, \quad D_5 = \frac{R_{2,SR}}{R_{2,TR}}$$

La discrimination entre TV et TSV peut être ensuite réalisée par divers types de classifieurs, en particulier par un classifieur linéaire ou par un classifieur neuronal.

Un premier mode de mise en oeuvre consiste à construire un classifieur linéaire dans l'espace 3D formé par exemple par les trois descripteurs $EQM$, $M_1$ et $D_1$ (cette méthode pouvant s'appliquer à d'autres descripteurs).

Un tel classifieur est caractérisé par l'équation du plan séparant dans cet espace les deux familles d'arythmies, TV et TSV.

Un plan séparateur robuste peut être obtenu par une minimisation des moindres carrés de la distance de chaque exemple au plan. L'équation du plan, caractérisé par son vecteur orthogonal A s'écrit :

$$\left\langle A, \begin{bmatrix} EQM \\ M_1 \\ D_1 \\ 1 \end{bmatrix} \right\rangle = 0 \quad \Leftrightarrow \quad A_1 EQM + A_2 M_1 + A_3 D_1 + A_4 = 0$$

A se calcule à partir des coordonnées de la base par :

$$A = (X^T X)^{-1} X^T Y$$

La matrice $X$ est la matrice contenant pour chacune des arythmies la valeur des trois descripteurs en colonnes, et une quatrième colonne de 1.

Cette matrice a la structure suivante, en supposant que l'on dispose d'une base de données de patients avec de 1 à N arythmies :

$$EQM(1)\ M_1(1)\ D_1(1)\ 1$$
$$EQM(2)\ M_1(2)\ D_1(2)\ 1$$

$$. \qquad ..$$
$$. \qquad ..$$
$$. \qquad ..$$

$$EQM(N)\ M_1(N)\ D_1(N)\ 1$$

La matrice $Y$ est le vecteur constitué de -1 lorsque le point correspond à une TSV, et de 1 si le point correspond à une TV.

Soit $O$ la matrice qui contient la nouvelle valeur des descripteurs en colonnes, et une quatrième colonne de 1 pour classifier une arythmie, et soit $z = A^t\ O$.

Si z est négatif l'arythmie est classée en TSV, si z est positif l'arythmie est classée en TV.

En variante, dans un souci de simplification des calculs et d'allègement de la charge de travail du processeur, il est possible d'appliquer la majeure partie des principes décrits dans ce qui précède sans recourir à l'analyse en composantes principales. Ainsi, les rapports $R_{1,SR}$ et $R_{2,SR}$, respectivement entre l'amplitude maximale et l'amplitude minimale du complexe de dépolarisation sur les axes BipV et UnipV à partir de l'EGM en rythme sinusal du patient, ainsi que les rapports $R_{1,TR}$ et $R_{2,TR}$ pour les battements en tachycardie peuvent être déterminés sans recourir à l'analyse en composantes principales. De la même manière peuvent être déterminés les maxima de corrélation $M_1$ et $M_2$ entre les tracés du battement sinusal et du battement en tachycardie, respectivement sur les axes BipV et UnipV. Sur la base des valeurs ainsi déterminées, il est alors possible de déduire les valeurs D4 et D5, pour poursuivre l'analyse sur la base de ces descripteurs, selon la description fournie dans les paragraphes précédents.

La base de données est évolutive et se complète en continu dans le dispositif, chaque arythmie venant soit s'ajouter soit prendre la place d'une arythmie de la base de données. Par ailleurs, le dispositif, implant ou programmateur, recalcule à intervalles réguliers la matrice $A$.

Le dispositif peut comporter des moyens complémentaires pour vérifier *a posteriori* la classification de l'arythmie, par exemple :

- si le dispositif détecte une TV par la méthode de la classification linéaire, il en avertit le patient par un signal sonore. Si la TV disparaît, la classification était erronée ;
- si le dispositif détecte une TSV qui se transforme en fibrillation ventriculaire, la classification était erronée ;
- si le dispositif fait une erreur de classification d'une arythmie, un enregistrement d'ECG tel un Holter permet de la détecter, le médecin l'indique par télémétrie au défibrillateur ;
- etc.

Le dispositif ayant ainsi avéré une erreur de classification d'une arythmie peut :

- soit ajouter l'arythmie à la base de données,
- soit remplacer une arythmie du même type dans la base de données puis recalculer la matrice $A$.

Une autre forme de réalisation peut, en variante, mettre en oeuvre un classifieur neuronal, ce qui permet notamment d'opérer au moyen d'un réseau adaptatif, au lieu d'un calcul purement mathématique.

Ce classifieur est construit dans l'espace 3D formé par exemple par les trois descripteurs $EQM$, $M_1$ et $D_1$ (cette méthode pouvant s'appliquer à d'autres descripteurs).

Un tel classifieur est caractérisé par l'équation du plan séparant dans cet espace les deux familles d'arythmies, TV et TSV. Soit :

$$y = f(W^T\ \Phi)$$

$$y = +1\ si\ W^T\ \Phi \geq 0\ ;$$

$$y=-1 \text{ si } \mathbf{W}^{T} \, \mathbf{\Phi} > 0$$

$W$ étant le vecteur constitué de poids appliqués à chaque descripteur ;

$\phi$ étant le vecteur contenant pour une arythmie en colonnes la valeur des trois descripteurs et le biais 1 (*EQM, M1, D1,* 1) ;

$y$ étant le prédicteur : si y est négatif l'arythmie est classée en TSV, si *y* est positif l'arythmie est classée en TV.

La valeur de *W* est déterminée par l'algorithme du gradient déterministe en appliquant la règle suivante :

*W* est initialisé ;

on fait l'apprentissage sur un ensemble d'arythmies préalablement classifiées et confirmées :

> s i la prédiction est bonne *W* n'est pas modifié
>
> s i pour une tachycardie supraventriculaire $\phi_n$ la prédiction est mauvaise, on soustrait à *W* la valeur de $\phi_n$ ;
>
> s i pour une tachycardie ventriculaire $\phi_m$ la prédiction est mauvaise, on additionne à *W* la valeur $\phi_m$.

La base de données est évolutive et se complète en continu dans le dispositif, chaque arythmie venant soit s'ajouter soit prendre la place d'une arythmie de la base de données. Par ailleurs, le dispositif, implant ou programmateur, refait à intervale régulier l'apprentissage de *W*.

Ici encore, le dispositif peut comporter de moyens complémentaires pour vérifier *a posteriori* la classification de l'arythmie, du même type que celles exposes plus haut, conduisant à un nouvel apprentissage de *W* à intervalles réguliers en cas d'erreurs de classification.

**Revendications**

**1.** Un dispositif médical actif, comprenant :

- des moyens de recueil de l'activité électrique du coeur, comprenant des moyens pour produire au moins deux composantes temporelles distinctes correspondant à deux signaux EGM d'électrogramme ventriculaire ;
- des moyens pour détecter la présence d'épisodes de tachycardie dans l'activité ainsi recueillie ; et
- des moyens de diagnostic de tachyarythmies ventriculaires, comprenant des moyens discriminateurs aptes à opérer une discrimination des tachycardies détectées entre tachycardies d'origine ventriculaire et tachycardies d'origine supraventriculaire,

dispositif dans lequel:

- les moyens de recueil comprennent des moyens pour produire lesdites au moins deux composantes temporelles distinctes à partir de deux signaux EGM distincts d'électrogramme ventriculaire respectifs provenant d'une même cavité du coeur et recueillis concurremment sur deux voies distinctes;
- les moyens de diagnostic comprennent des moyens d'analyse bidimensionnelle, aptes à déterminer, à partir des variations de l'une desdites composantes temporelles en fonction de l'autre, une caractéristique 2D représentative d'un battement cardiaque ; et
- les moyens discriminateurs comprennent des moyens pour comparer :

  · une première caractéristique 2D courante, représentative d'un battement en tachycardie (TSV ; TV), issue desdits au moins deux signaux EGM recueillis lors d'un épisode de tachycardie, avec
  · une deuxième caractéristique 2D de référence, représentative d'un battement en rythme sinusal (RS) issue desdits au moins deux signaux EGM, recueillis hors épisodes de tachycardie.

**2.** Le dispositif de la revendication 1, dans lequel les moyens d'analyse bidimensionnelle sont des moyens aptes à déterminer ladite caractéristique 2D, à partir des variations de l'une desdites composantes temporelles en fonction de l'autre, ces variations étant considérées sur une fraction temporelle du battement cardiaque incluse dans une fenêtre temporelle (W) incluant le complexe QRS de ce battement cardiaque.

**3.** Le dispositif de la revendication 1, dans lequel les moyens de diagnostic comprennent des moyens pour déterminer ladite caractéristique 2D de référence à partir d'une pluralité de battements cardiaques successifs moyennés.

**4.** Le dispositif de la revendication 3, dans lequel les moyens de diagnostic comprennent des moyens pour détecter des battements non représentatifs dans ladite pluralité de battements cardiaques, et exclure ces battements non représentatifs lors de la détermination de la caractéristique 2D de référence.

**5.** Le dispositif de la revendication 4, dans lequel les moyens pour détecter les battements non représentatifs comprennent des moyens d'analyse morphologique de ladite pluralité de battements cardiaques par corrélation croisée.

**6.** Le dispositif de la revendication 5, dans lequel les moyens d'analyse morphologique de ladite pluralité de battements cardiaques comprennent en outre des moyens de sélection par *clustering* des battements non représentatifs.

**7.** Le dispositif de la revendication 1, dans lequel les moyens discriminateurs comprennent des moyens pour caractériser lesdites caractéristiques 2n courante et de référence par au moins un descripteur géométrique ($\vec{e}_T$, c), et pour comparer ensuite lesdites caractéristiques 2D courante et de référence par le(s) descripteur(s) ainsi déterminé(s).

**8.** Le dispositif de la revendication 7, dans lequel ledit descripteur géométrique est le vecteur tangent unitaire ($\vec{e}_T$) à la caractéristique 2D, considéré en une pluralité de points.

**9.** Le dispositif de la revendication 8, dans lequel les moyens discriminateurs comprennent des moyens pour évaluer un angle moyen entre les vecteurs tangents unitaires respectifs de la caractéristique 2D courante et de la caractéristique 2D de référence.

**10.** Le dispositif de la revendication 7, dans lequel ledit descripteur géométrique est la norme du vecteur tangent à la caractéristique 2D, considéré en une pluralité de points.

**11.** Le dispositif de la revendication 10, dans lequel les moyens discriminateurs comprennent des moyens pour évaluer un coefficient de corrélation entre les normes des vecteurs tangents respectifs de la caractéristique 2D courante et de la caractéristique 2D de référence.

**12.** Le dispositif de la revendication 7, dans lequel ledit descripteur géométrique est la courbure (c) de la caractéristique 2D, considérée en en une pluralité de points.

**13.** Le dispositif de la revendication 12, dans lequel les moyens discriminateurs comprennent des moyens pour évaluer un coefficient de corrélation entre les courbures respectives de la caractéristique 2D courante et de la caractéristique 2D de référence.

**14.** Le dispositif de la revendication 1, dans lequel les moyens d'analyse bidimensionnelle comprennent des moyens de détermination de repère, aptes à déterminer un repère de référence orthonormé dont l'un des axes correspond à l'axe principal du coeur.

**15.** Le dispositif de la revendication 14, dans lequel les moyens de détermination de repère comprennent des moyens d'analyse en composantes principales appliquée à un signal EGM sinusal recueilli hors épisodes de tachycardie.

**16.** Le dispositif de la revendication 14, comprenant des moyens pour appliquer auxdites première et deuxième caractéristiques 2D un changement de repère, de leur repère primitif vers ledit repère de référence.

**17.** Le dispositif de la revendication 16, dans lequel lesdits moyens de diagnostic comprennent des moyens d'analyse en composantes principales aptes à produire des premiers paramètres descripteurs de la morphologie desdites première et deuxième caractéristiques 2D.

**18.** Le dispositif de la revendication 17, dans lequel lesdits premiers paramètres descripteurs sont des paramètres parmi : première et deuxième valeur propre de la matrice de covariance ; vecteurs propres de la matrice de covariance associés à chacune de ces valeurs propres ; orientation des axes principal et secondaire ; rapport entre les amplitudes extrêmes de signal sur chacune des voies ; aire circonscrite par la caractéristique 2D.

**19.** Le dispositif de la revendication 14, dans lequel lesdits moyens de diagnostic comprennent des moyens aptes à produire une première et une deuxième composante unidimensionnelle par projection de chacune desdites première et deuxième caractéristiques 2D sur l'un des axes dudit repère de référence.

**20.** Le dispositif de la revendication 19, dans lequel lesdits moyens de diagnostic comprennent des moyens aptes à produire des seconds paramètres descripteurs de la morphologie desdites première et deuxième composantes unidimensionnelles.

**21.** Le dispositif de la revendication 20, dans lequel lesdits seconds paramètres descripteurs sont des paramètres parmi : hauteur maximale du signal ; hauteur minimale du signal ; largeur du signal.

**22.** Le dispositif de la revendication 14, dans lequel lesdits moyens de diagnostic comprennent des moyens d'analyse d'intercorrélation entre lesdites première et deuxième caractéristiques 2D.

**23.** Le dispositif de la revendication 22, dans lequel lesdits moyens d'analyse d'intercorrélation comprennent des moyens d'analyse de distribution bidimensionnelle entre coefficients de corrélation et valeurs propres de la matrice de covariance d'une analyse en composantes principales.

**24.** Le dispositif de la revendication 23, dans lequel lesdits moyens d'analyse d'intercorrélation comprennent des moyens d'analyse de distribution tridimensionnelle, aptes à définir, pour au moins un paramètre descripteur de la morphologie desdites première et deuxième caractéristiques 2D, un plan discriminateur entre tachycardies d'origine ventriculaire et tachycardies d'origine supraventriculaire.

**25.** Le dispositif de la revendication 24, dans lequel lesdits moyens d'analyse de distribution tridimensionnelle comprennent des moyens classifleurs linéaires.

**26.** Le dispositif de la revendication 24, dans lequel lesdits moyens d'analyse de distribution tridimensionnelle comprennent des moyens classifieurs à réseau neuronal adaptatif.

**27.** Le dispositif de la revendication 14, dans lequel lesdits moyens de diagnostic sont essentiellement dépourvus de moyens d'analyse en composantes principales.

**28.** Le dispositif de la revendication 27, dans lequel lesdits moyens de diagnostic comprennent des moyens pour déterminer des rapports entre l'amplitude maximale et l'amplitude minimale d'un complexe de dépolarisation pour chacune desdites deux composantes temporelles distinctes, respectivement pour lesdits battements en rythme sinusal et en tachycardie.

**29.** Le dispositif de la revendication 27, dans lequel lesdits moyens de diagnostic comprennent des moyens pour déterminer des maxima de corrélation entre lesdites caractéristiques 2D respectives desdits battements en rythme sinusal et en tachycardie.

**30.** Le dispositif de la revendication 1, dans lequel lesdits deux signaux EGM distincts sont :

- l'un, un signal de composante unipolaire (UnipV) recueilli entre le boitier du dispositif et une électrode proximale ou distale, et
- l'autre, un signal de composante bipolaire (BipV) recueilli entre une électrode proximale et une électrode distale.

**Claims**

**1.** An active medical device comprising:

- means for collecting the electrical activity of the heart, comprising means for producing at least two distinct time components corresponding to two ventricular electrogram EGM signals;
- means for detecting the presence of tachycardia episodes in the activity thus collected; and
- means for diagnosing ventricular tachyarrhythmia, comprising discriminating means capable of making a discrimination of the detected tachycardias between tachycardias of ventricular origin and tachycardias of supraventricular origin,

a device in which:

- the collection means comprise means for producing the said at least two distinct time components based on two respective distinct electrogram EGM signals originating from one and the same heart cavity and collected concurrently on two distinct channels;

- the diagnostic means comprise two-dimensional analysis means capable of determining, based on the variations of one of the said time components as a function of the other, a 2D characteristic representative of a heartbeat; and
- the discriminating means comprise means for comparing:

    . a first current 2D characteristic, representative of a tachycardia beat (TSV; TV), originating from the said at least two EGM signals collected during an episode of tachycardia, with

    . a second reference 2D characteristic, representative of a sinus rhythm (RS) beat originating from the said at least two EGM signals, collected outside episodes of tachycardia.

2. The device of Claim 1, in which the two-dimensional analysis means are means capable of determining the said 2D characteristic based on variations in one of the said time components as a function of the other, these variations being considered over a time fraction of the heartbeat included in a time window (W) including the QRS complex of this heartbeat.

3. The device of Claim 1, in which the diagnostic means comprise means for determining the said reference 2D characteristic based on a plurality of averaged successive heartbeats.

4. The device of Claim 3, in which the diagnostic means comprise means for detecting unrepresentative beats in the said plurality of heartbeats, and for excluding these unrepresentative beats during the determination of the reference 2D characteristic.

5. The device of Claim 4, in which the means for detecting the unrepresentative beats comprise means of morphological analysis of the said plurality of heartbeats by cross-correlation.

6. The device of Claim 5, in which the means of morphological analysis of the said plurality of heartbeats also comprise selection means by clustering of the unrepresentative beats.

7. The device of Claim 1, in which the discriminating means comprise means for characterizing the said current and reference 2D characteristics by at least one geometric descriptor ($\overline{e}T$, c), and for subsequently comparing the said current and reference 2D characteristics via the descriptor(s) thus determined.

8. The device of Claim 7, in which the said geometric descriptor is the unitary tangent vector ($\overline{e}T$) to the 2D characteristic, considered at a plurality of points.

9. The device of Claim 8, in which the discriminating means comprise means for evaluating an average angle between the respective unitary tangent vectors of the current 2D characteristic and of the reference 2D characteristic.

10. The device of Claim 7, in which the said geometric descriptor is the norm of the tangent vector to the 2D characteristic, considered at a plurality of points.

11. The device of Claim 10, in which the discriminating means comprise means for evaluating a coefficient of correlation between the norms of the respective tangent vectors of the current 2D characteristic and of the reference 2D characteristic.

12. The device of Claim 7, in which the said geometric descriptor is the curvature (c) of the 2D characteristic, considered at a plurality of points.

13. The device of Claim 12, in which the discriminating means comprise means for evaluating a coefficient of correlation between the respective curvatures of the current 2D characteristic and of the reference 2D characteristic.

14. The device of Claim 1, in which the two-dimensional analysis means comprise coordinate determination means capable of determining an orthonormal reference coordinate of which one of the axes corresponds to the main axis of the heart.

15. The device of Claim 14, in which the coordinate determination means comprise main-component analysis means applied to a sinus EGM signal collected outside episodes of tachycardia.

16. The device of Claim 14, comprising means for applying to the said first and second 2D characteristics a change of coordinate, from their primitive coordinate to the said reference coordinate.

17. The device of Claim 16, in which the said diagnostic means comprise means for analysis in main components capable of producing first descriptor parameters of the morphology of said first and second 2D characteristics.

18. The device of Claim 17, in which the said first descriptor parameters are parameters amongst: a first and second specific value of the covariance matrix; specific vectors of the covariance matrix associated with each of these specific values; orientation of main and secondary axes; relationship between the extreme signal amplitudes on each of the channels; area circumscribed by the 2D characteristic.

19. The device of Claim 14, in which the said diagnostic means comprise means capable of producing a first and a second one-dimensional component by projection of each of the said first and second 2D characteristics onto one of the axes of the said reference coordinate.

20. The device of Claim 19, in which the said diagnostic means comprise means capable of producing second descriptor parameters of the morphology of the
said first and second one-dimensional components.

21. The device of Claim 20, in which the said second descriptor parameters are parameters amongst: maximum height of the signal; minimum height of the signal; width of the signal.

22. The device of Claim 14, in which the said diagnostic means comprise means for analysing intercorrelation between the said first and second 2D characteristics.

23. The device of Claim 22, in which the said intercorrelation analysis means comprise means for analysing two-dimensional distribution between coefficients of correlation and specific values of the covariance matrix of a main-component analysis.

24. The device of Claim 23, in which the said intercorrelation analysis means comprise means for analysing three-dimensional distribution, capable of defining, for at least one descriptor parameter of the morphology of the said first and second 2D characteristics, a discriminating plane between tachycardias of ventricular origin and tachycardias of supraventricular origin.

25. The device of Claim 24, in which the said means for analysing three-dimensional distribution comprise linear classifying means.

26. The device of Claim 24, in which the said means for analysing three-dimensional distribution comprise classifying means with adaptive neuronal network.

27. The device of Claim 14, in which the said diagnostic means are essentially bereft of main-component analysis means.

28. The device of Claim 27, in which the said diagnostic means comprise means for determining relationships between the maximum amplitude and the minimum amplitude of a depolarization complex for each of the said two distinct time components, respectively for the said sinus rhythm and tachycardia beats.

29. The device of Claim 27, in which the said diagnostic means comprise means for determining maxima of correlation between the said respective 2D characteristics of the said sinus rhythm and tachycardia beats.

30. The device of Claim 1, in which the said two distinct EGM signals are:

   - one, a unipolar-component signal (UnipV) collected between the casing of the device and a proximal or distal electrode, and
   - the other, a bipolar-component signal (BipV) collected between a proximal electrode and a distal electrode.

**Patentansprüche**

1.  Aktive medizinische Vorrichtung, die enthält:

    - Einrichtungen zum Auffangen der elektrischen Aktivität des Herzens, die Einrichtungen enthalten, um mindestens zwei unterschiedliche zeitliche Komponenten zu erzeugen, die zwei EGM-Signalen eines ventrikulären Elektrogramms entsprechen;
    - Einrichtungen, um das Vorhandensein von Tachykardie-Episoden in der so aufgefangenen Aktivität zu erfassen; und
    - Einrichtungen zur Diagnose von ventrikulären Tachyarrhythmien, die Unterscheidungseinrichtungen enthalten, welche in der Lage sind, eine Unterscheidung der erfassten Tachykardien zwischen Tachykardien ventrikulären Ursprungs und Tachykardien supraventrikulären Ursprungs durchzuführen,

    Vorrichtung, bei der:

    - die Auffangeinrichtungen Einrichtungen enthalten, um die mindestens zwei unterschiedlichen zeitlichen Komponenten ausgehend von zwei unterschiedlichen EGM-Signalen eines ventrikulären Elektrogramms zu erzeugen, die von einer gleichen Kavität des Herzens stammen und gleichzeitig in zwei verschiedenen Kanälen aufgefangen werden;
    - die Diagnoseeinrichtungen Einrichtungen zur zweidimensionalen Analyse enthalten, die ausgehend von den Änderungen einer der zeitlichen Komponenten in Abhängigkeit von der anderen eine 2D-Charakteristik bestimmen können, die für einen Herzschlag repräsentativ ist; und
    - die Unterscheidungseinrichtungen Einrichtungen enthalten, um zu vergleichen:

        • eine für einen Tachykardie-Schlag (TSV; TV) repräsentative laufende erste 2D-Charakteristik, die von den mindestens zwei EGM-Signalen stammt, die während einer Tachykardie-Episode aufgefangen werden, mit
        • einer für einen Schlag im Sinusrhythmus (RS) repräsentativen zweiten 2D-Bezugscharakteristik, die von den mindestens zwei EGM-Signalen stammt, die außerhalb von Tachykardie-Episoden aufgefangen werden.

2.  Vorrichtung nach Anspruch 1, bei der die Einrichtungen zur zweidimensionalen Analyse Einrichtungen sind, die die 2D-Charakteristik ausgehend von den Änderungen einer der zeitlichen Komponenten in Abhängigkeit von der anderen bestimmen können, wobei diese Änderungen über einen zeitlichen Bruchteil des Herzschlags berücksichtigt werden, der in einem Zeitfenster (W) enthalten ist, das den QRS-Komplex dieses Herzschlags enthält.

3.  Vorrichtung nach Anspruch 1, bei der die Diagnoseeinrichtungen Einrichtungen enthalten, um die 2D-Bezugscharakteristik ausgehend von mehreren aufeinanderfolgenden gemittelten Herzschlägen zu bestimmen.

4.  Vorrichtung nach Anspruch 3, bei der die Diagnoseeinrichtungen Einrichtungen enthalten, um nicht repräsentative Schläge unter den mehreren Herzschlägen zu erfassen, und diese nicht repräsentativen Schläge bei der Bestimmung der 2D-Bezugscharakteristik auszuschließen.

5.  Vorrichtung nach Anspruch 4, bei der die Einrichtungen zur Erfassung der nicht repräsentativen Schläge Einrichtungen zur morphologischen Analyse der mehreren Herzschläge durch Kreuzkorrelation enthalten.

6.  Vorrichtung nach Anspruch 5, bei der die Einrichtungen zur morphologischen Analyse der mehreren Herzschläge außerdem Auswahleinrichtungen der nicht repräsentativen Schläge durch Clustering enthalten.

7.  Vorrichtung nach Anspruch 1, bei der die Unterscheidungseinrichtungen Einrichtungen enthalten, um die laufende 2D-Charakteristik und die 2D-Bezugscharakteristik durch mindestens einen geometrischen Deskriptor ($\vec{e_T}$, c) zu charakterisieren, und um anschließend die laufende 2D-Charakteristik und die 2D-Bezugscharakteristik durch den (die) so bestimmten Deskriptor(en) zu vergleichen.

8.  Vorrichtung nach Anspruch 7, bei der der geometrische Deskriptor der Einheits-Tangentenvektor ($\vec{e_T}$) der 2D-Charakteristik ist, betrachtet an mehreren Punkten.

9.  Vorrichtung nach Anspruch 8, bei der die Unterscheidungseinrichtungen Einrichtungen enthalten, um einen mittleren Winkel zwischen den Einheits-Tangentenvektoren der laufenden 2D-Charakteristik bzw. der 2D-Bezugscharakteristik zu ermitteln.

10. Vorrichtung nach Anspruch 7, bei der der geometrische Deskriptor die Norm des Tangentenvektors der 2D-Charakteristik ist, betrachtet an mehreren Punkten.

11. Vorrichtung nach Anspruch 10, bei der die Unterscheidungseinrichtungen Einrichtungen enthalten, um einen Korrelationskoeffizienten zwischen den Normen der Tangentenvektoren der laufenden 2D-Charakteristik bzw. der 2D-Bezugscharakteristik zu ermitteln.

12. Vorrichtung nach Anspruch 7, bei der der geometrische Deskriptor die Krümmung (c) der 2D-Charakteristik ist, betrachtet in mehreren Punkten.

13. Vorrichtung nach Anspruch 12, bei der die Unterscheidungseinrichtungen Einrichtungen enthalten, um einen Korrelationskoeffizienten zwischen den Krümmungen der laufenden 2D-Charakteristik bzw. der 2D-Bezugscharakteristik zu ermitteln.

14. Vorrichtung nach Anspruch 1, bei der die Einrichtungen zur zweidimensionalen Analyse Einrichtungen zur Bestimmung eines Koordinatensystems enthalten, die fähig sind, ein orthonormiertes Bezugs-Koordinatensystem zu bestimmen, von dem eine der Achsen der Hauptachse des Herzens entspricht.

15. Vorrichtung nach Anspruch 14, bei der die Einrichtungen zur Bestimmung eines Koordinatensystems Einrichtungen zur Hauptkomponentenanalyse enthalten, die an ein EGM-Sinussignal angewendet wird, das außerhalb von Tachykardie-Episoden aufgefangen wird.

16. Vorrichtung nach Anspruch 14, die Einrichtungen enthält, um an die erste und zweite 2D-Charakteristik eine Änderung des Koordinatensystems von ihrem ursprünglichen Koordinatensystem zum Bezugs-Koordinatensystem anzuwenden.

17. Vorrichtung nach Anspruch 16, bei der die Diagnoseeinrichtungen Einrichtungen zur Hauptkomponentenanalyse enthalten, die fähig sind, erste Deskriptorparameter der Morphologie der ersten und zweiten 2D-Charakteristik zu erzeugen.

18. Vorrichtung nach Anspruch 17, bei der die ersten Deskriptorparameter Parameter sind unter: erster und zweiter Eigenwert der Kovarianzmatrix; Eigenvektoren der Kovarianzmatrix, die jedem dieser Eigenwerte zugeordnet sind; Ausrichtung der Haupt- und Sekundärachse; Verhältnis zwischen den Signal-Endamplituden auf jedem der Kanäle; von der 2D-Charakteristik umschriebener Bereich.

19. Vorrichtung nach Anspruch 14, bei der die Diagnoseeinrichtungen Einrichtungen enthalten, die fähig sind, eine erste und eine zweite eindimensionale Komponente durch Projektion jeder der ersten und zweiten 2D-Charakteristiken auf eine der Achsen des Bezugs-Koordinatensystems zu erzeugen.

20. Vorrichtung nach Anspruch 19, bei der die Diagnoseeinrichtungen Einrichtungen enthalten, die fähig sind, zweite Deskriptorparameter der Morphologie der ersten und zweiten eindimensionalen Komponenten zu erzeugen.

21. Vorrichtung nach Anspruch 20, bei der die zweiten Deskriptorparameter Parameter sind unter: maximale Höhe des Signals; minimale Höhe des Signals; Breite des Signals.

22. Vorrichtung nach Anspruch 14, bei der die Diagnoseeinrichtungen Einrichtungen zur Interkorrelationsanalyse zwischen der ersten und der zweiten 2D-Charakteristik enthalten.

23. Vorrichtung nach Anspruch 22, bei der die Einrichtungen zur Interkorrelationsanalyse Einrichtungen zur Analyse einer zweidimensionalen Verteilung zwischen Korrelationskoeffizienten und Eigenwerten der Kovarianzmatrix einer Hauptkomponentenanalyse enthalten.

24. Vorrichtung nach Anspruch 23, bei der die Einrichtungen zur Interkorrelationsanalyse Einrichtungen zur Analyse einer dreidimensionalen Verteilung enthalten, die fähig sind, für mindestens einen Deskriptorparameter der Morphologie der ersten und der zweiten 2D-Charakteristiken eine Diskriminatorebene zwischen Tachykardien ventrikulären Ursprungs und Tachykardien supraventrikulären Ursprungs zu definieren.

25. Vorrichtung nach Anspruch 24, bei der die Einrichtungen zur Analyse einer dreidimensionalen Verteilung lineare Klassifizierer-Einrichtungen enthalten.

26. Vorrichtung nach Anspruch 24, bei der die Einrichtungen zur Analyse einer dreidimensionalen Verteilung Klassifizierer-Einrichtungen mit adaptivem neuronalem Netz enthalten.

27. Vorrichtung nach Anspruch 14, bei der die Diagnoseeinrichtungen im Wesentlichen keine Einrichtungen zur Hauptkomponentenanalyse enthalten.

28. Vorrichtung nach Anspruch 27, bei der die Diagnoseeinrichtungen Einrichtungen enthalten, um Verhältnisse zwischen der maximalen Amplitude und der minimalen Amplitude eines Depolarisationskomplexes für jede der zwei verschiedenen zeitlichen Komponenten für die Schläge im Sinusrhythmus bzw. in Tachykardie zu bestimmen.

29. Vorrichtung nach Anspruch 27, bei der die Diagnoseeinrichtungen Einrichtungen enthalten, um Korrelationsmaxima zwischen den 2D-Charakteristiken der Schläge im Sinusrhythmus bzw. in Tachykardie zu bestimmen.

30. Vorrichtung nach Anspruch 1, bei der die zwei verschiedenen EGM-Signale sind:

- das eine ein unipolares Komponentensignal (UnipV), das zwischen dem Gehäuse der Vorrichtung und einer proximalen oder distalen Elektrode aufgefangen wird, und
- das andere ein bipolares Komponentensignal (BipV), das zwischen einer proximalen Elektrode und einer distalen Elektrode aufgefangen wird.

FIG_1

FIG_2

## FIG_3

## FIG_4

## FIG_5

## FIG_6

## FIG_7

Nouvelle onde R détectée sur Vbip — 10

8 ondes R stockées — 12

8 fenêtres de 80 ms centrees sur chaque onde R de Vbip — 14

Non

Rythme est lent? — 16

Oui

Non

Besoin de créer ou actualiser le battement de référence? — 18

Oui

Parmi les 8 stockés choix des battements représentatifs — 20

22 — Moyenne point à point sur Vbip

Moyenne point à point sur Vuni — 22'

Vectogramme du battement de référence — 24

26 — Vecteurs tangents unitaires eTréf

Courbure ponderée et normalsee Cpnréf — 26'

FIG_8

FIG_9

FIG_10

## FIG_11

Nouvelle onde R détectée sur Vbip — 30

8 ondes R stockées — 32

8 fenêtres de 80ms centrées sur chaque onde R de Vbip — 34

Non

TV confirmée par l'analyse rythmologique? — 36

Oui

8 vectogrammes — 38

40 — Vecteurs tangents unitaires $e_{Ti}$

42 — Angle moyen entre $e_{Tréf}$ et $e_{Ti}$ < seuil ?

Non

Courbure pondérée et normalisée $C_{pni}$ — 46

Coefficient de correlation entre $C_{pnréf}$ et $C_{pni}$ > f(bpm)? — 48

Oui

Oui          Non

44 — TSV          TV — 50

Parmi les 8 battements, combien de TV? — 52

> 5          < 3          sinon

TV          TSV          pas de majorité

54          56          58

## FIG_12

a) Vectogramme RS

b) Vectogramme TSV

c) Angles entre chaque vecteur tangent unitaire et le vecteur (1,0)

f) Angles entre chaque vecteur tangent unitaire et le vecteur (1,0)

d) Courbure brute

g) Courbure brute

e) Courbure pondérée et normalisée

h) Courbure pondérée et normalisée

i) Angle moyen entre les vecteurs tangents unitaires

bpm

j) Coefficient de corrélation entre les courbures

bpm

# FIG_13

a) Vectogramme RS

b) Vectogramme TV

c) Angles entre chaque vecteur tangent unitaire et le vecteur (1,0)

f) Angles entre chaque vecteur tangent unitaire et le vecteur (1,0)

d) Courbure brute

g) Courbure brute

e) Courbure pondérée et normalisée

h) Courbure pondérée et normalisée

i) Angle moyen entre les vecteurs tangents unitaires

bpm

j) Coefficient de corrélation entre les courbures

bpm

## FIG_14

FIG_15

FIG_16

## FIG_17

## FIG_18

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0626182 A1
- EP 0838235 A1
- EP 0813888 A1
- EP 1208873 A1
- US 20050159781 A1
- US 7149569 B1
- WO 0069517 A1
- US 20030083587 A